(19) Europäisches Patentamt
European Patent Office
Office européen des brevets

(11) **EP 3 526 711 B1**

(12) **EUROPEAN PATENT SPECIFICATION**

(45) Date of publication and mention
of the grant of the patent:
**29.03.2023 Bulletin 2023/13**

(21) Application number: **16791320.1**

(22) Date of filing: **11.10.2016**

(51) International Patent Classification (IPC):
***G16B 50/50*** *(2019.01)*

(52) Cooperative Patent Classification (CPC):
**G16B 50/00; G16B 30/00; G16B 30/10;
G16B 50/50**

(86) International application number:
**PCT/EP2016/074307**

(87) International publication number:
**WO 2018/068829 (19.04.2018 Gazette 2018/16)**

(54) **METHOD AND APPARATUS FOR COMPACT REPRESENTATION OF BIOINFORMATICS DATA**

VERFAHREN UND VORRICHTUNG ZUR KOMPAKTEN DARSTELLUNG VON
BIOINFORMATIKDATEN

PROCÉDÉ ET APPAREIL DESTINÉS À UNE REPRÉSENTATION COMPACTE DE DONNÉES
BIOINFORMATIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**21.08.2019 Bulletin 2019/34**

(73) Proprietor: **Genomsys SA
1015 Lausanne (CH)**

(72) Inventors:
• **ZOIA, Giorgio
1007 Lausanne (CH)**
• **RENZI, Daniele
1004 Lausanne (CH)**

(74) Representative: **Metroconsult Srl
Foro Bonaparte 51
20121 Milano (IT)**

(56) References cited:
**US-A1- 2015 227 686**

• **Anonymous: "SAM", , 11 March 2015
(2015-03-11), XP002771304, Retrieved from the
Internet:
URL:https://web.archive.org/web/2015031104
5750/http://davetang.org/wiki/tiki-index.p
hp?page=SAM [retrieved on 2017-06-22]**
• **Anonymous: "CRAM format specification
(version 3.0)", , 8 September 2016 (2016-09-08),
XP002771305, Retrieved from the Internet:
URL:https://samtools.github.io/hts-specs/C
RAMv3.pdf [retrieved on 2017-06-22]**

**Description**

**TECHNICAL FIELD**

This disclosure provides a novel computer-implemented method for the compression of genome sequence data, a novel method for decompression of a genomic stream compressed according to the said method for compression, a novel genomic encoder for the compression of genome sequence data and a novel genomic decoder for de decompression of a genomic stream compressed by the said genomic encoder. The said computer-implemented method for the compression of genome sequence data may reduce the utilized storage space and improves access performance by providing new functionality that are not available with known prior art methods of representation.

**BACKGROUND**

An appropriate representation of genome sequencing data is fundamental to enable efficient genomic analysis applications such as genome variants calling and all other analysis performed with various purposes by processing the sequencing data and metadata.

Human genome sequencing has become affordable by the emergence of high-throughput low cost sequencing technologies. Such opportunity opens new perspectives in several fields ranging from the diagnosis and treatment of cancer to the identification of genetic illnesses, from pathogen surveillance for the identification of antibodies to the creation of new vaccines, drugs and customization of personalized treatments.

Hospitals, genomic analysis providers, bioinformatics and large biological data storage centers are looking for affordable, fast, reliable and interconnected genomic information processing solutions which could enable scaling genomic medicine to a world-wide scale. Since one of the bottleneck in the sequencing process has become data storage, methods for representing genome sequencing data in a compressed form are increasingly investigated.

The most used genome information representations of sequencing data are based on zipping FASTQ and SAM formats. The objective is to compress the traditionally used file formats (respectively FASTQ and SAM for non-aligned and aligned data). Such files are constituted by plain text characters and are compressed, as mentioned above, by using general purpose approaches such as LZ (from Lempel and Ziv, the authors who published the first versions) schemes (the well-known zip, gzip etc). When general purpose compressors such as gzip are used, the result of compression is usually a single blob of binary data. The information in such monolithic form results quite difficult to archive, transfer and elaborate particularly when like in the case of high throughput sequencing the volume of data are extremely large. The BAM format is characterized by poor compression performance due to the focus on compression of the inefficient and redundant SAM format rather than on extracting the actual genomic information conveyed by SAM files and due to the adoption of general purpose text compression algorithms such as gzip rather than exploiting the specific nature of each data source (the genomic data itself).

A more sophisticated approach to genomic data compression that is less used, but more efficient than BAM is CRAM. CRAM provides more efficient compression for the adoption of differential encoding with respect to an existing reference (it partially exploits the data source redundancy), but it still lacks features such as incremental updates, support for streaming and selective access to specific classes of compressed data.

These approaches generate poor compression ratios and data structures that are difficult to navigate and manipulate once compressed. Downstream analysis can be very slow due to the necessity of handling large and rigid data structures even to perform simple operation or to access selected regions of the genomic dataset. CRAM relies on the concept of the CRAM record. Each CRAM record encodes a single mapped or unmapped reads by encoding all the elements necessary to reconstruct it.

Document "CRAM format specification (version 3.0)" - hereinafter "CRAM" - published on Septembers, 2016 describes the CRAM 3.0 format and its objectives, being for example full compatibility with SAM and effortless transition to CRAM from using SAM files. Said document is a reference based compression toolkit, employing GZIP. The coding structure of this document is based on GZIP as a monolithic file (see e.g. Chapter 14 of CRAM).

CRAM performs indexing by means of the creation of an external file which contains a tree-based structure for the retrieval of compressed blocks. Since CRAM suffers from the same limitations of SAM and it is silent on the notion of any data classification according to the result of the mapping process, the only indexing available in CRAM relies on the notion of genomic position. Furthermore, in formats such as SAM and CRAM where data classification is not present, it is not possible to index compressed data blocks according to the result of the mapping process apart from the generic criteria "mapped" or "unmapped" Summarizing, the compression scheme proposed in said document has a mediocre performance.

CRAM has the following drawbacks:

1. For CRAM, data indexing is out of the scope of the specification (see section 12 of CRAM specification v 3.0) and it's implemented as a separate file. Conversely the approach of the invention described in this document employs a data indexing method that is integrated with the encoding process and indexes are embedded in the encoded bit stream.

2. In CRAM all core data blocks can contain any type of mapped reads (perfectly matching reads, reads with substitutions only, reads with insertions or deletions (also referred to as "indels")). There is no notion of classification and grouping of reads in classes according to the result of mapping with respect to a reference sequence

3. In the present invention there is no notion of record encapsulating each read because the data needed to reconstruct each read is scattered among several data containers called "layers". This enables more efficient access to set of reads with specific biological characteristics (e.g. reads with substitutions, but without "indels", or perfectly mapped reads) without the need of decoding each (block of) read(s) to inspect its features.

4. In a CRAM record each type of data is denoted by a specific flag. In the present invention there is no notion of flag denoting data because this is intrinsically defined by the "layer" the data belongs to. This implies a largely reduced number of symbols to be used and a consequent reduction of the information source entropy which results into a more efficient compression. This is due to the fact that the use of different "layers" enables the encoder to reuse the same symbol across each layer with different meanings. In CRAM each flag must always have the same meaning as there is no notion of contexts and each CRAM record can contain any type of data.

5. In CRAM substitutions, insertions and deletions are expressed according to different syntaxes, while the proposed approach uses a single alphabet and encoding for substitutions, insertions and deletions. This makes the encoding and decoding process simpler and produces a lower entropy source model which coding yields high compression bitstreams.

The present invention aims at compressing genomic sequences by organizing and partitioning data so that the redundant information to be coded is minimized and features such as selective access and support for incremental updates are enabled.

One of the aspects of the presented approach is the definition of classes of data and metadata to be encoded separately and to be structured in different layers. The most important improvements of this approach with respect to existing methods consist in:

1. an increase of compression performance due to the reduction of the information source entropy constituted by providing an efficient model for each class of data or metadata;
2. the possibility of performing selective accesses to portions of the compressed data and metadata for any further processing purpose;
3. the possibility to incrementally (without the need of re-encoding) update encoded data and metadata with new sequencing data and/or metadata and/or new analysis results.

Document US 2015/0227686 A relates to an apparatus and a processor-implemented method, wherein the method includes aligning a reference genome with a plurality of DNA sequences. Each of the plurality of DNA sequences has a respective plurality of based. Further, the method includes classifying and sorting the plurality of read sequences based on respective numbers of mismatched bases within the plurality of read sequences to obtain a plurality of rearranged DNA sequences.

The latest document describes the implementation of an aligner based on the Burrows Wheeler Transform. The output of the alignment process is a set of records describing how the reads map on a reference genome. The output format of such process is the textual SAM usually ordered by mapping position on a reference sequence (e.g. chromosome). The US-document is silent on any classification of reads according to the result of the mapping process and all reads are shuffled when they are sorted according to the mapping position. Therefore when an end user needs to extract only perfectly mapping reads or any other category of reads, the only way to find them is to decompress the entire file and perform post processing on the textual SAM output.

## BRIEF DESCRIPTION OF THE DRAWINGS

Figure 1 shows how the position of the mapped reads pairs are encoded in the pos layer as difference from the absolute position of the first mapped read.

Figure 2 shows how two reads in a pair can come from the two DNA strands.

Figure 3 shows how the reverse complement of read 2 will be encoded if strand 1 is used as reference.

Figure 4 shows the four possible combinations of reads composing a reads pair and the respective encoding in the rcomp layer.

Figure 5 shows how to calculate the pairing distance in case of constant reads length for three read pairs.

Figure 6 show how the pairing errors encoded in the pair layer enable the decoder to reconstruct the correct read pairing using the encoded MPPPD.

Figure 7 shows the encoding of a pairing distance when a read is mapped on a difference reference than its mate. In this case additional descriptors are added to the pairing distance. One is a signaling flag, the second is a reference identifier and then the pairing distance.

Figure 8 shows the encoding of N mismatches in a nmis layer.

Figure 9 shows a mapped read pair which presents substitutions with respect to a reference sequence.

Figure 10 shows how to calculate the positions of substitutions either as absolute or differential values.

Figure 11 shows how to calculate the symbols encoding substitutions types when no IUPAC codes are used. The symbols represent the distance - in a circular substitution vector - between the molecule present in the read and the one present on the reference at that position.

Figure 12 shows how to encode the substitutions into the snpt layer.

Figure 13 shows how to calculate substitution codes when IUPAC ambiguity codes are used.

Figure 14 shows how the snpt layer is encoded when IUPAC codes are used.

Figure 15 shows how for reads of class I the substitution vector used is the same as for class M with the addition of special codes for insertions of the symbols A, C, G, T, N.

Figure 16 shows some examples of encoding of mismatches and indels in case of IUPAC ambiguity codes. The substitution vector is much longer in this case and therefore the possible calculated symbols are more than in the case of five symbols.

Figure 17 shows a different source model for mismatches and indels where each layer contains the position of the mismatches or inserts of a single type. In this case no symbols are encoded for the mismatch or indel type.

Figure 18 shows an example of mismatches and indels encoding. When no mismatches or indels of a given type are present for a read, a 0 is encoded in the corresponding layer. The 0 acts as reads separator and terminator in each layer.

Figure 19 shows how a modification in the reference sequence can transform M reads in P reads. This operation can reduce the information entropy of the data structure especially in case of high coverage.

Figure 20 shows a genomic encoder 2010 according to one embodiment of this invention.

Figure 21 shows a genomic decoder 218 according to one embodiment of this invention.

## SUMMARY

The aforementioned problems present in the prior art are solved by the present invention as claimed in the set of appended claims.

In one aspect, a computer-implemented method for the compression of genome sequence data , said genome sequence data comprising reads of sequences of nucleotides,
said method comprising the steps of:

aligning said reads to a reference sequence thereby creating aligned reads;
classifying said aligned reads into different classes at least comprising:

- a first class: when said aligned reads match said one or more reference sequences without any error;
- a second class: when said aligned reads match a region in said one or more reference sequences with a number of mismatches constituted by a number of positions in which the sequencing machine was not able to call any base;
- a third class: when said aligned reads match a region in said one or more reference sequences with a number of mismatches constituted by a number of positions in which the sequencing machine was not able to call any base or it called a different base than the one reported in the reference genome.
- a fourth class: when said aligned reads match a region in said one or more reference sequences with a number of mismatches constituted by a number of positions in which the sequencing machine was not able to call any base, or it called a different base than the one reported in the reference genome and by the presence of insertions or deletions or clipped nucleotides;
- a fifth class: when said aligned reads do not find any valid mapping on said one or more reference sequences according to specified alignment constraints,;

thereby creating classes of aligned reads;
encoding said classified and aligned reads as a multiplicity of layers of syntax elements defined by descriptors that univocally represent genome sequence reads, said descriptors comprising for said first class at least the start position on the reference sequence, a flag signaling if the read has to be considered as a reverse complement versus the reference, a distance to the mate pair in case of paired reads, the value of the length in case of the sequencing technology produces variable length reads, said descriptors comprising for said second class at least the descriptors of said first class and a mismatch position for each mismatch, said descriptors comprising for said third class the descriptors of said second class and a mismatch position and a mismatch type for each mismatch, said descriptors comprising for said fourth class the descriptors of said first class and, mismatch type for each mismatch, and the clipped bases,;

wherein encoding said classified aligned reads as a multiplicity of layers of syntax elements comprises selecting said syntax elements comprising said descriptors according to said classes of aligned reads,

wherein the encoding of said classified aligned reads as a multiplicity of layers of syntax elements is adapted according to the statistical properties of the data carried by the layer,

wherein the encoding of said classified aligned reads as a multiplicity of layers of syntax elements comprising said descriptors associates a specific source model and a specific entropy coder to each layer, the source model being characterized by the definition of the syntax elements emitted by each source, the definition of an associated probability model and the definition of the associated entropy coder,

wherein there is decomposition of the sequence read data and metadata into homogeneous layers of said descriptors in order to obtain distinct information sources with reduced information entropy.

In another aspect, a method for the decompression of a genomic stream compressed according to a method for compression according to the present invention, said method comprising the steps of:

parsing and decoding the compressed genomic stream into genomic layers of syntax elements,

expanding said genomic layers into classified reads of sequences of nucleotides,

selectively decoding using class decoders said classified reads of sequences of nucleotides and merging the resulton one or more reference sequences so as to produce uncompressed reads of sequences of nucleotides.

A further aspect, a genomic encoder 2010 for the compression of genome sequence data 209, said genome sequence data 209 comprising reads of sequences of nucleotides, said genomic encoder 2010 comprising:

an aligner unit 201, configured to align said reads to one or more reference sequences thereby creating aligned reads,

a data classification unit 204, configured to classify said aligned reads according to matching accuracy degrees with the one or more reference sequences thereby creating classes of aligned reads into different classes at least comprising:

- a first class: when said aligned reads match said one or more reference sequence without any error;

- a second class: when said aligned reads match a region in said one or more reference sequences with a number of mismatches constituted by a number of positions in which the sequencing machine was not able to call any base;

- a third class: when said aligned reads match a region in said one or more reference sequences with a number of mismatches constituted by a number of positions in which the sequencing machine was not able to call any base or it called a different base than the one reported in the reference genome.

- a fourth class: when said aligned reads match a region in said one or more reference sequences with a number of mismatches constituted by a number of positions in which the sequencing machine was not able to call any base, or it called a different base than the one reported in the reference genome and by the presence of insertions or deletions or clipped nucleotides;

- a fifth class: when said aligned reads do not find any valid mapping on said one or more reference sequences according to specified alignment constraints,

Thereby creating classes of aligned reads;

one or more layers encoding units 205-207, configured to encode said classified aligned reads as layers of syntax elements by selecting said syntax elements according to said classes of aligned reads, wherein said descriptors comprise for said first class at least the start position on the reference sequence, a flag signaling if the read has to be considered as a reverse complement versus the reference, a distance to the mate pair in case of paired reads, the value of the length in case of the sequencing technology produces variable length reads, said descriptors comprising for said second class at least the descriptors of said first class and a mismatch position for each mismatch, said descriptors comprising for said third class the descriptors of said second class and a mismatch position and a mismatch type for each mismatch, said descriptors comprising for said fourth class the descriptors of said first class and a mismatch type for each mismatch and the clipped bases;
entropy coder units (2012-2014) for entropy coding said layers of syntax elements,
wherein the encoding of said classified aligned reads as a multiplicity of layers of syntax elements is adapted according to the statistical properties of the data carried by the layer,
wherein the encoding of said classified aligned reads as a multiplicity of layers of syntax elements comprising said descriptors associates a specific source model and a specific entropy coder to each layer, the source model being characterized by the definition of the syntax elements emitted by each source, the definition of an associated probability model and the definition of the associated entropy coder,
wherein there is decomposition of the sequence read data and metadata into homogeneous layers of said descriptors in order to obtain distinct information sources with reduced information entropy..

In another aspect, a genomic decoder 218 for the decompression of a genomic stream 211, compressed by a genomic encoder as described hereinabove and hereinafter, said genomic decoder 218 comprising:

Parsing and decoding means 210, 212-214 configured to parse said compressed genomic stream into genomic layers of syntax elements 215,

one or more layer decoders 216-217, configured to decode the genomic layers into classes of data and further configured to process said genomic layers into classified reads of sequences of nucleotides 2111,

genomic data classes decoders 213 configured to selectively decode said classified reads of sequences of nucleotides and configured to merge the result on one or more reference sequences so as to produce uncompressed reads of sequences of nucleotides.

## DETAILED DESCRIPTION

The genomic or proteomic sequences referred to in this invention include, for example, and not as a limitation, nucleotide sequences, Deoxyribonucleic acid (DNA) sequences, Ribonucleic acid (RNA), and amino acid sequences. Although the description herein is in considerable detail with respect to genomic information in the form of a nucleotide sequence, it will be understood that the methods and systems for compression can be implemented for other genomic or proteomic sequences as well, albeit with a few variations, as will be understood by a person skilled in the art.

Genome sequencing information is generated by High Throughput Sequencing (HTS) machines in the form of sequences of nucleotides (a. k. a. bases) represented by strings of letters from a defined vocabulary. The smallest vocabulary is represented by five symbols: {A, C, G, T, N} representing the 4 types of nucleotides present in DNA namely Adenine, Cytosine, Guanine, and Thymine. In RNA Thymine is replaced by Uracil (U). N indicates that the sequencing machine was not able to call any base and so the real nature of the position is undetermined. In case the IUPAC ambiguity codes are adopted by the sequencing machine, the alphabet used for the symbols is (A, C, G, T, U, W, S, M, K, R, Y, B, D, H, V, N or -).

The nucleotides sequences produced by sequencing machines are called **"reads".** Sequence reads can be between a few dozens to several thousand nucleotides long. Some technologies produce sequence reads in **pairs** where one read is from one DNA strand and the second is from the other strand. In genome sequencing the term **coverage** is used to express the level of redundancy of the sequence data with respect to a **reference sequence.** For example, to reach a coverage of 30x on a human genome (3.2 billion bases long) a sequencing machine shall produce a total of 30 x 3.2 billion bases so that in average each position in the reference is "covered" 30 times.

Throughout this disclosure, a **reference sequence** is any sequence on which the nucleotides sequences produced by sequencing machines are aligned/mapped. One example of sequence could actually be a **reference genome,** a sequence assembled by scientists as a representative example of a species' set of genes. For example GRCh37, the Genome Reference Consortium human genome (build 37) is derived from thirteen anonymous volunteers from Buffalo, New York. However, a reference sequence could also consist of a synthetic sequence conceived to merely improve the compressibility of the reads in view of their further processing.

Sequencing devices can introduce errors in the sequence reads such as

1. Use of a wrong symbol (i.e. representing a different nucleic acid) to represent the nucleic acid actually present in the sequenced sample; this is usually called "substitution error" (mismatch);

2. Insertion in one sequence read of additional symbols that do not refer to any actually present nucleic acid; this is usually called "insertion error";

3. Deletion from one sequence read of symbols that represent nucleic acids that are actually present in the sequenced sample; this is usually called "deletion error";

4. Recombination of one or more fragments into a single fragment which does not reflect the reality of the originating sequence;

The term "coverage" is used in literature to quantify the extent to which a reference genome or part thereof can be covered by the available sequence reads. Coverage is said to be:

• partial (less than 1X) when some parts of the reference genome are not mapped by any available sequence read.

• single (1X) when all nucleotides of the reference genome are mapped by one and only one symbol present in the sequence reads.

• multiple (2X, 3X, NX) when each nucleotide of the reference genome is mapped multiple times.

This invention aims at defining a genomic information representation format where the relevant information is efficiently accessible and transportable and the weight of the redundant information is reduced.
The main aspects of the present disclosure are:

1 The classification of the sequence reads in different classes according to the results of the alignment with respect to the reference sequences in order to enable selective access to encoded data according to criteria related to the alignment results and to matching accuracy.

2 The decomposition of the sequence read data and metadata into homogeneous layers of in order to obtain distinct information sources with reduced information entropy.

3 The possibility of modeling each separated source with distinct source model adapted to each statistical characteristics including the possibility of changing the source model within each class of reads and layer for each accessible data units (access units). Adoption of the appropriate context adaptive probability models and associated entropy coders according to the statistical properties of each source model.

4 The definition of correspondences and dependencies among the layers to enable selective access to the data without the need to decode all the layers if not all information is needed

5 Coding each sequence data class and associated metadata layers with respect to a reference sequence that can be modified so as to reduce the entropy of data classes and layers information sources. After a first encoding based on a reference, sequence the detected mismatches can be used to "adapt/modify" the reference sequence in order to further reduce the overall information entropy. This process that can be performed iteratively as long as the reduction of information entropy results relevant.

In the following, each of the above aspects will be further described.

**Main file header**

**Classification of the sequence reads**

The sequence reads generated by sequencing machines may be classified in the context of the present disclosure into five different "classes" according to the results of the alignment with respect to one or more given reference sequences.
When aligning a DNA sequence of nucleotides with respect to a reference sequence five are the possible results:

1. A region in the reference sequence is found to match the sequence read without any error (perfect mapping).

Such sequence of nucleotides will be referenced to as "perfectly matching read" or denoted as "Class P".

2. A region in the reference sequence is found to match the sequence read with a number of mismatches constituted by a number of positions in which the sequencing machine was not able to call any base (or nucleotide). Such mismatches are denoted by an "N". Such sequences will be referenced to as "N mismatching reads" or "Class N".

3. A region in the reference sequence is found to match the sequence read with a number of mismatches constituted by a number of positions in which the sequencing machine was not able to call any base (or nucleotide) OR a different base than the one reported in the reference genome has been called. Such type of mismatch is called Single Nucleotide Variation (SNV) or Single Nucleotide Polymorphism (SNP). The sequence will be referenced to as "M mismatching reads" or "Class M".

4. A fourth class is constituted by sequencing reads presenting a mismatch type that includes the same mismatches of class M plus the presence of insertions or deletions (a.k.a. *indels*). Insertions are represented by a sequence of one or more nucleotides not present in the reference, but present in the read sequence. In literature when the inserted sequence is at the edges of the sequence it is referred to as "soft clipped" (i.e. the nucleotides are not matching the reference but are kept in the aligned reads contrarily to "hard clipped" nucleotides which are discarded). Keeping or discarding nucleotides is typically a user's decisions implemented as a configuration of the aligning tool. Deletion are "holes" (missing nucleotides) in the aligned read with respect to the reference. Such sequences will be referenced to as "I mismatching reads" or "Class I".

5. A fifth class includes all reads that do now find any valid mapping on the reference genome according to the specified alignment constraints. Such sequences are said to be Unmapped and belonging to "Class U".

The remaining unmapped reads with respect to a reference sequence can be assembled into a single sequence using de-novo assembly algorithms. Once a newly assembled reference sequence has been created unmapped reads can be further mapped with respect to it and be classified in one of the 4 classes P, N, M and I.

## Decomposition of the information necessary to represent sequence reads into layers of descriptors

Once the classification of reads is completed with the definition of the Classes, further processing consists in defining a set of distinct syntax elements which represent the remaining information enabling the reconstruction of the DNA read sequence when represented as being mapped on a given reference sequence. The data structure of these syntax elements requires the storage of global parameters and metadata to be used by the decoding engine. These data are structured in a ***main header*** described in the table below.

**Table 1- Main Header structure.**

| Element | Type | Description |
|---|---|---|
| **Unique ID** | Byte array | Unique identifier for the encoded content |
| **Version** | Byte array | Major + Minor version of the encoding algorithm |
| **Header Size** | Integer | Size in bytes of the entire encoded content |
| **Reads Length** | Integer | Size of reads in case of constant reads length. A special value (e.g. 0) is reserved for variable reads length |
| **Ref count** | Integer | Number of reference sequences used |
| **Access Units counters** | Byte array (e.g. integers) | Total Number of encoded Access Units per reference sequence |
| **Ref ids** | Byte array | Unique identifiers for reference sequences |
| **Master index table** *Alignment positions of first read in each block (Access Unit). I.e. smaller position of the first read on the reference genome per each block of the 4 classes 1 per pos class (4) per reference* | Byte array (e.g. integers) | This is a multidimensional array supporting random access to Access Units. |

A DNA segment referred to a given reference sequence can be fully expressed by:

- The starting position on the reference sequence (pos)
- A flag signaling if the read has to be considered as a reverse complement versus the reference (rcomp).
- A distance, to the mate pair in case of paired reads (pair).
- The value of the read length in case of the sequencing technology produces variable length reads (len). In case of constant reads length the read length associated to each reads can obviously be omitted and can be stored in the main file header.
- For each mismatch:

  o Mismatch position (*nmis* for class N, *snpp* for class M, and *indp* for class I)
  o Mismatch type (not present in class N, *snpt* in class M, *indt* in class I)

- Flags indicating specific characteristics of the sequence read such as

  o template having multiple segments in sequencing
  o each segment properly aligned according to the aligner
  o unmapped segment
  o next segment in the template unmapped
  o signalization of first or last segment
  o quality control failure
  o PCR or optical duplicate
  o secondary alignment
  o supplementary alignment

- Optional soft clipped nucleotides string when present (*indc* in class I)

This classification creates groups of descriptors (syntax elements) that can be used to univocally represent genome sequence reads. The table below summarizes the syntax elements needed for each class of aligned reads.

**Table 2 - Defined layers per class of data.**

|       | P | N | M | I |
|-------|---|---|---|---|
| pos   | X | X | X | X |
| pair  | X | X | X | X |
| rcomp | X | X | X | X |
| flags | X | X | X | X |
| rlen  | X | X | X | X |
| nmis  |   | X |   |   |
| snpp  |   |   | X |   |
| snpt  |   |   | X |   |
| indp  |   |   |   | X |
| indt  |   |   |   | X |
| indc  |   |   |   | X |

Reads belonging to class P are characterized and can be perfectly reconstructed by only a position, a reverse complement information and an offset between mates in case they have been obtained by a sequencing technology yielding mated pairs, some flags and a read length.
The next section further details how these descriptors are defined.

**Position descriptor layer**

In the position (pos) layer only the mapping position of the first encoded read is stored as absolute value on the reference sequence. All the other position descriptors assume a value expressing the difference with respect to the previous position. Such modeling of the information source defined by the sequence of read position descriptors is in general characterized by a reduced entropy particularly for sequencing processes generating high coverage results.

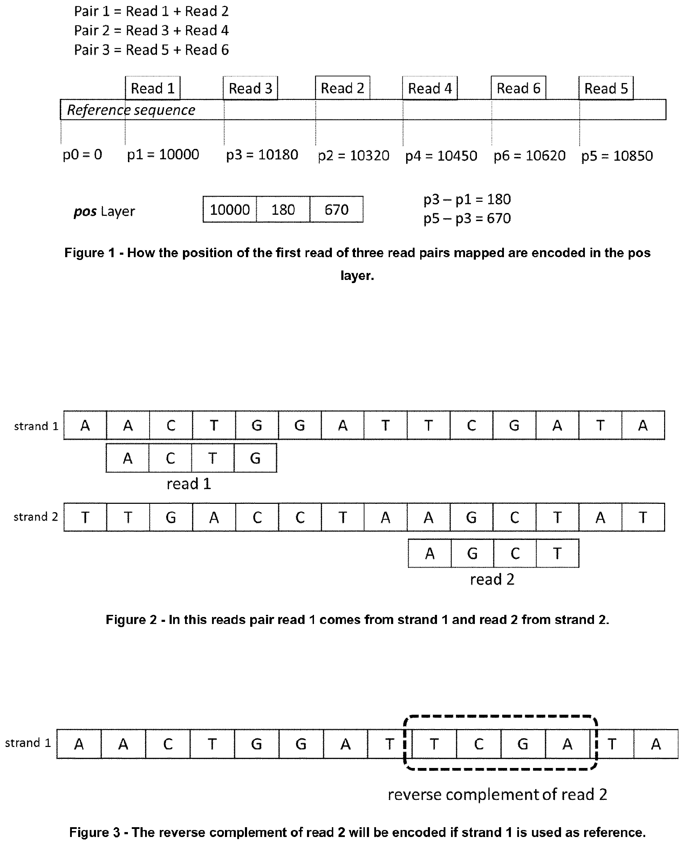

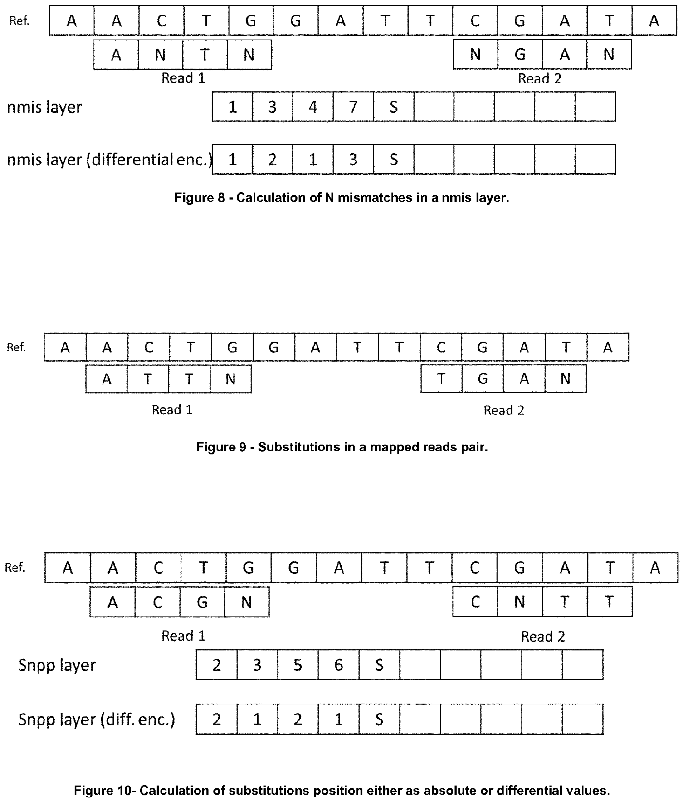

For example, figure 1 shows how after describing the starting position of the first alignment as position "10000" on the reference sequence, the position of the second read starting at position 10180 is described as "180". With high coverages (> 50x) most of the descriptors of the position vector will present very high occurrences of low values such as 0 and 1 and other small integers. Figure 9 shows how the positions of three read pairs are described in a **pos** Layer.

**Reverse complement descriptor layer**

Each read of the read pairs produced by sequencing technologies can be originated from either genome strands of the sequenced organic sample. However, only one of the two strands is used as reference sequence. Figure 2 shows how in a reads pair one read (read 1) can come from one strand and the other (read 2) can come from the other.

When the strand 1 is used as reference sequence, read 2 can be encoded as *reverse complement* of the corresponding fragment on strand 1. This is shown in figure 3.

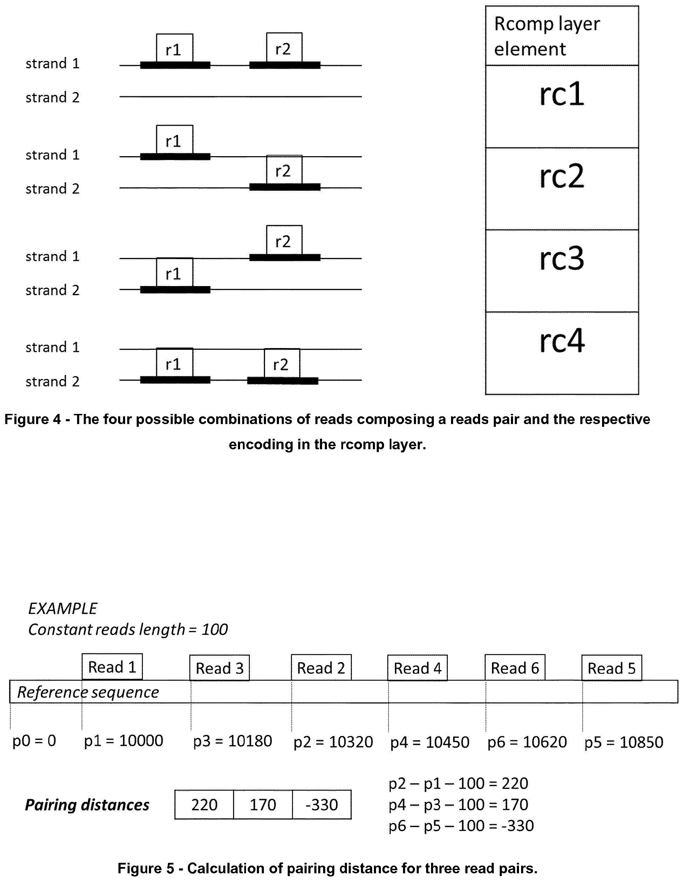

In case of coupled reads, four are the possible combinations of direct and reverse complement mate pairs. This is shown in figure 4. The rcomp layer encodes the four possible combinations .

The same encoding is used for the reverse complement information of reads belonging to classes N, M, P and I. In order to enable selective access to the different data classes, the reverse complement information of reads belonging to the four classes are encoded in different layers as depicted in Table 2.

**Pairing information descriptor layer**

The pairing descriptor is stored in the pair layer. Such layer stores descriptors encoding the information needed to reconstruct the originating reads pairs when the employed sequencing technology produces reads by pairs. Although at the date of the disclosure of the invention the vast majority of sequencing data is generated by using a technology generating paired reads, it is not the case of all technologies. This is the reason for which the presence of this layer is not necessary to reconstruct all sequencing data information if the sequencing technology of the genomic data considered does not generate paired reads information.

**Definitions:**

- **mate pair:** read associated to another read in a read pair (e.g. Read 2 is the mate pair of Read 1 in the previous example)
- **pairing distance:** number of nucleotide positions on the reference sequence which separate one position in the first read (pairing anchor, e.g. last nucleotide of first read) from one position of the second read (e.g. the first nucleotide of the second read)
- **most probable pairing distance (MPPD):** this is the most probable pairing distance expressed in number of nucleotide positions.
- **position pairing distance (PPD):** the PPD is a way to express a pairing distance in terms of the number of reads separating one read from its respective mate present in a specific position descriptor layer.
- **most probable position pairing distance (MPPPD):** is the most probable number of reads separating one read from its mate pair present in a specific position descriptor layer.
- **position pairing error (PPE):** is defined as the difference between the MPPD or the MPPPD and the actual position of the mate.
- **pairing anchor:** position of first read last nucleotide in a pair used as reference to calculate the distance of the mate pair in terms of number of nucleotide positions or number of read positions.

Figure 5 shows how the pairing distance among read pairs is calculated.

The pair descriptor layer is the vector of pairing errors calculated as number of reads to be skipped to reach the mate pair of the first read of a pair with respect to the defined decoding pairing distance.

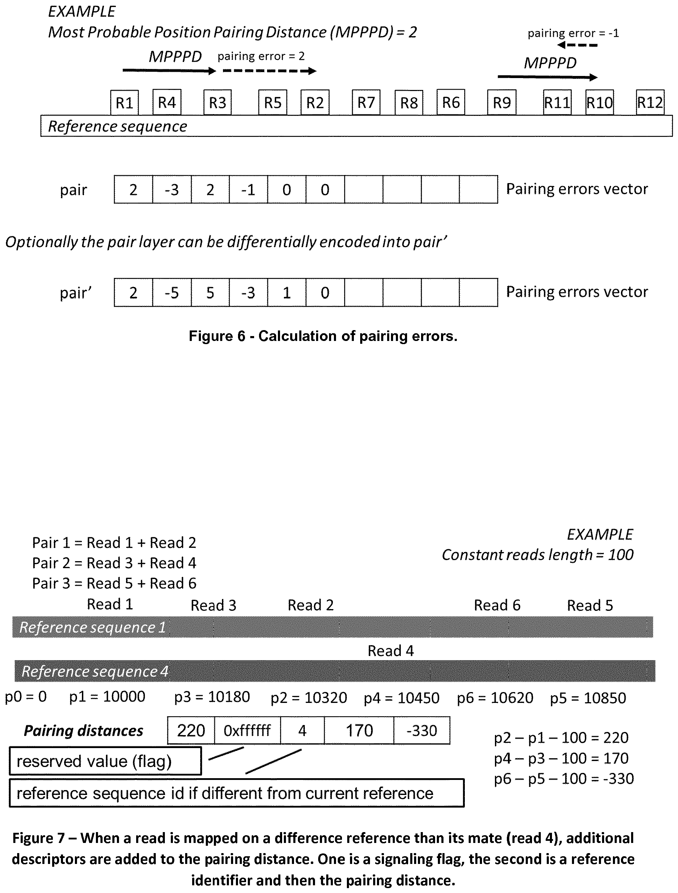

Figure 6 shows an example of how pairing errors are calculated, both as absolute value and as differential vector (characterized by lower entropy for high coverages).

The same descriptors are used for the pairing information of reads belonging to classes N, M, P and I. In order to enable the selective access to the different data classes, the pairing information of reads belonging to the four classes are encoded in different layer as depicted in.

**Pairing information in case of reads mapped on different reference sequences**

In the process of mapping sequence reads on a reference sequence it is not uncommon to have the first read in a pair mapped on one reference sequence (e.g. chromosome 1) and the second on a different reference sequence (e.g. chromosome 4). In this case the pairing information described above has to be integrated by additional information related to the reference sequence used to map one of the reads. This is achieved by coding

1. A reserved value (flag) indicating that the pair is mapped on two different sequences (different values indicate if read1 or read2 are mapped on the sequence that is not currently encoded)
2. An unique reference identifier referring to the reference identifiers encoded in the main header structure as described Table 1.
3. The third element contains the mapping information on the reference identified at point 2 and expressed as offset with respect to the last encoded position.

The figure 7 provides an example of this scenario.
In figure 7, since Read 4 is not mapped on the currently encoded reference sequence, the genomic encoder signals this information by crafting additional descriptors in the pair layer. In the example shown below Read 4 of pair 2 is mapped on reference no. 4 while the currently encoded reference is no. 1. This information is encoded using 3 components:

1) One special reserved value is encoded as pairing distance (in this case 0xffffff)
2) A second descriptor provides a reference ID as listed in the main header (in this case 4)
3) The third element contains the mapping information on the concerned reference (170).

**Mismatch descriptors for class N reads**

Class N includes all reads in which only mismatches constituted by "N" are present at the place of an A, C, G or T base call. All other bases of the read perfectly match the reference sequence.
Figure 8 shows how:

the positions of "N" in read 1 are coded as

- absolute position in read 1 or
- as differential position with respect to the previous "N" in the same read.

the positions of "N" in read 2 are encoded as

- absolute position in read 2 + read 1 length or
- differential position with respect to the previous N

In the **nmis** layer, the coding of each reads pair is terminated by a special "separator" symbol.
Figure 8 shows how "N" mismatches (where, at a given mapping position, a "N" is present in a read instead of an actual base in the reference sequence) are encoded only as a the position of the mismatch

1. with respect to the beginning of the read or
2. with respect to the previous mismatch (differential encoding)

**Descriptors coding Substitutions (Mismatches or SNPs), Insertions and Deletions**

A substitution is defined as the presence, in a mapped read, of a different nucleotide base with respect to the one that is present in the reference sequence at the same position.
Figure 9 shows examples of substitutions in a mapped read pair. Each substitution is encoded as "position" (snpp layer) and "type" (snpt layer). Depending on the statistical occurrence of substitutions, insertion or deletion, different source models of the associated descriptors can be defined and the generated symbols coded in the associated layer.

**Source model 1: Substitutions as Positions and Types**

**Substitutions Positions Descriptors**

A substitution position is calculated like the values of the nmis layer, i.e.
In read 1 substitutions are encoded

- as absolute position in read 1 or
- as differential position with respect to the previous substitution in the same read In read 2 substitutions are encoded
- as absolute position in read 2 + read 1 length or
- as differential position with respect to the previous substitution

Figure 10 shows how substitutions (where, at a given mapping position, a symbol in a read is different from the symbol in the reference sequence) are coded as

1. the position of the mismatch

- with respect to the beginning of the read or
- with respect to the previous mismatch (differential encoding)

2. the type of mismatch represented as a code calculated as described in Figure 10

In the snpp layer, the coding of each reads pair is terminated by a special "separator" symbol.

**Substitutions Types Descriptors**

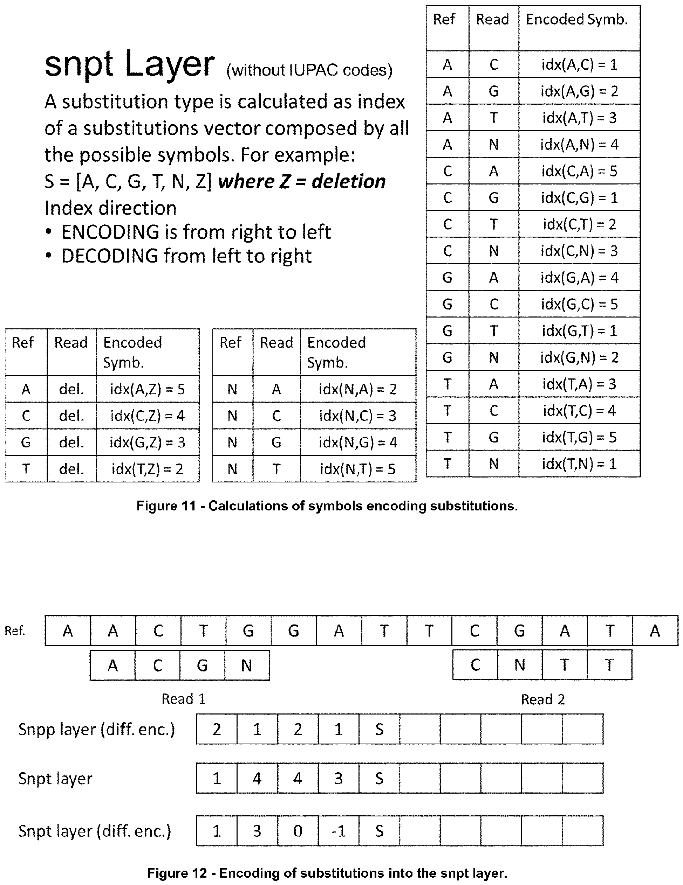

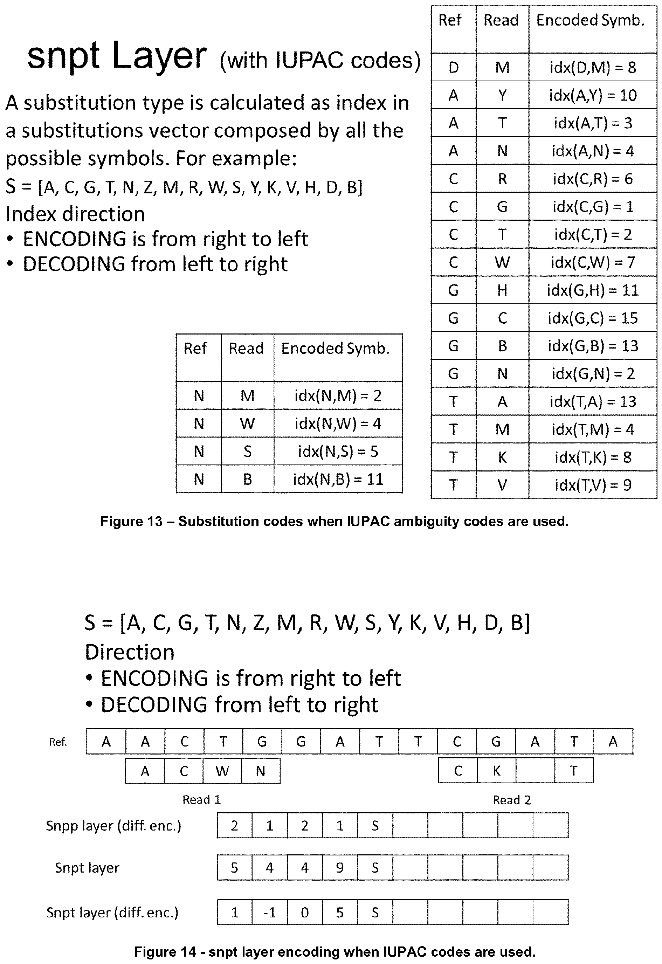

For class M (and I as described in the next sections), mismatches are coded by an index (moving from right to left) from the actual symbol present in the reference to the corresponding substitution symbol present in the read {A, C, G, T, N, Z}. For example if the aligned read presents a C instead of a T which is present at the same position in the reference, the mismatch index will be denoted as "4". The decoding process reads the encoded syntax element, the nucleotide at the given position on the reference and moves from left to right to retrieve the decoded symbol. E.g. a "2" received for a position where a G is present in the reference will be decoded as "N". Figure 11 shows all the possible substitutions and the respective encoding symbols. Obviously different and context adaptive probability models can be assigned to each substitution index according to the statistical properties of each substitution type for each data class to minimize the entropy of the descriptors.
In case of adoption of the IUPAC ambiguity codes the substitution mechanism results to be exactly the same however the substitution vector is extended as: S = {A, C, G, T, N, Z, M, R, W, S, Y, K, V, H, D, B}.
Figure 12 provides an example of encoding of substitutions types in the ***snpt*** layer.
Some examples of substitutions encoding when IUPAC ambiguity codes are adopted are provided in Figure 13. A further example of substitution indexes is provided in Figure 14.

**Cooding of insertions and deletions**

For class I, mismatches and deletions are coded by an indexes (moving from right to left) from the actual symbol present in the reference to the corresponding substitution symbol present in the read: {A, C, G, T, N, Z}. For example if the aligned read presents a C instead of a T present at the same position in the reference, the mismatch index will be "4". In case the read presents a deletion where a A is present in the reference, the coded symbol will be "5". The decoding process reads the coded syntax element, the nucleotide at the given position on the reference and moves from left to right to retrieve the decoded symbol. E.g. a "3" received for a position where a G is present in the reference will be decoded as "Z".
Inserts are coded as 6, 7, 8, 9, 10 respectively for inserted A, C, G, T, N.

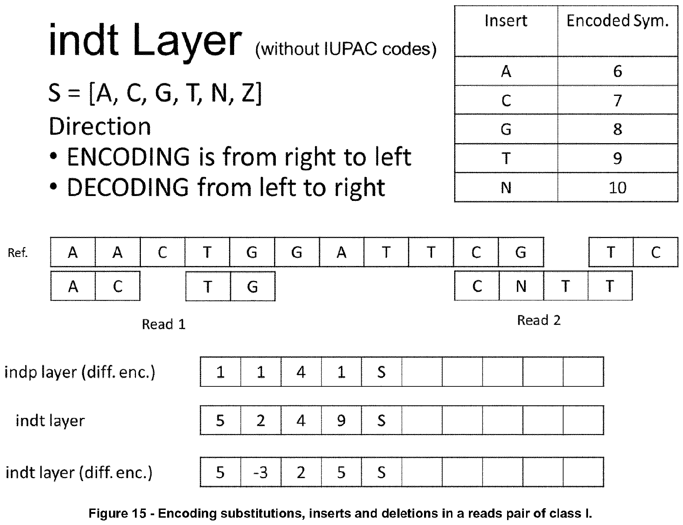

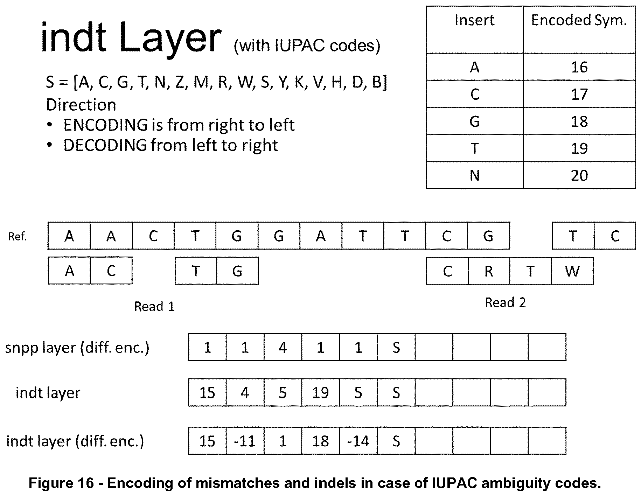

Figure 15 shows an example of how to encode substitutions, inserts and deletions in a reads pair of class I. In order to support the entire set of IUPAC ambiguity codes, the substitution vector S= {A, C, G, T, N, Z} shall be replaced by S = {A, C, G, T, N, Z, M, R, W, S, Y, K, V, H, D, B} as described in the previous paragraph for mismatches. In this case the insertion codes need to have different values, namely 16, 17, 18, 19, 20 in case the substitution vector has 16 elements. The mechanism is illustrated in Figure 16.

**Source model 2: One layer per substitution type and indels**

For some data statistics a different coding model from the one described in the previous section can be developed for substitutions and indels resulting into a source with lower entropy. Such coding model is an alternative to the techniques described above for mismatches only and for mismatches and indels.

In this case one data layer is defined for each possible substitution symbol (5 without IUPAC codes, 16 with IUPAC codes), plus one layer for deletions and 4 more layers for insertions. For simplicity of the explanation, but not as a limitation for the application of the model, the following description will focus on the case where no IUPAC codes are supported.

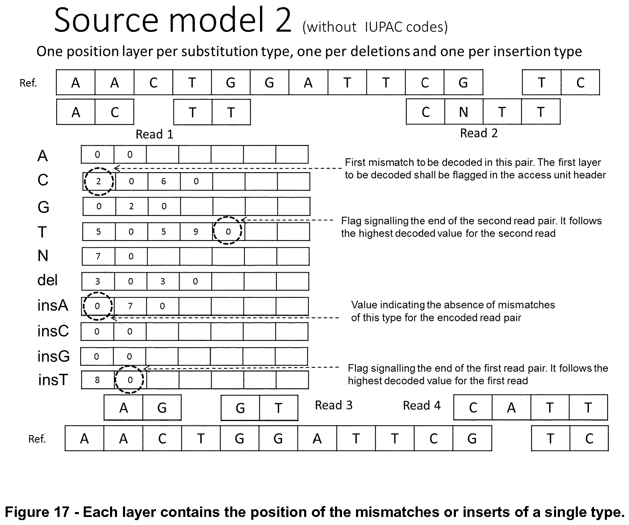

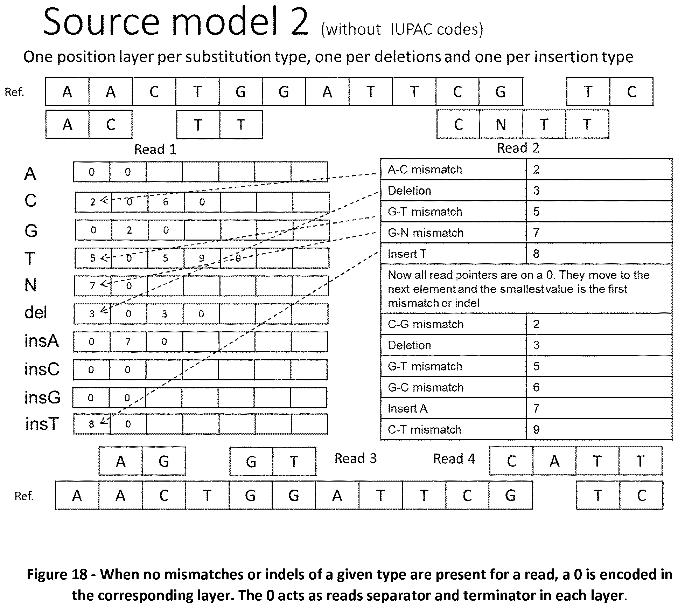

Figure 17 shows how each layer contains the position of the mismatches or inserts of a single type. If no mismatches or inserts for that type is present in the encoded read pair, a 0 is encoded in the corresponding layer. To enable the decoder to start the decoding process for the layers described in this section, the header of each access units contains a flag signaling the first layer to be decoded. In the example of figure 18 the first element to be decoded is position 2 in the C layer. When no mismatches or indels of a given type are present in a read pair, a 0 is added to the corresponding layers. On the decoding side, when the decoding pointer for each layer points to a value of 0, the decoding process moves to the next read pair.

**Encoding additional signaling flags**

Each data class introduced above (P, M, N, I) may require the encoding of additional information on the nature of the encoded reads. This information may be related for example to the sequencing experiment (e.g. indicating a probability of duplication of one read) or can express some characteristic of the read mapping (e.g. first or second in pair). In the context of this invention this information is encoded in a separate layer for each data class. The main advantage of such approach is the possibility to selectively access this information only in case of need and only in the required reference sequence region. Other examples of the use of such flags are:

- read paired
- read mapped in proper pair
- read or mate unmapped
- read or mate from reverse strand
- first/second in pair
- not primary alignment
- read fails platform/vendor quality checks
- read is PCR or optical duplicate
- supplementary alignment

**Adaptation of the reference sequences**

The mismatches encoded for classes N, M and I can be used to create "modified references" to be used to re-encode reads in the N, M or I layer (with respect to the first reference sequence, R0) as p reads with respect to the "adapted" genome R1. For example if we denote with $r\_in^M$ the ith read of class M containing mismatches with respect to the reference genome n, then after "adaptation" we could have $r\_in^M = r\_{(i(n+1))}^P$ with A(Refn)=Refn+1 where A is the transformation from reference sequence n to reference sequence n + 1.

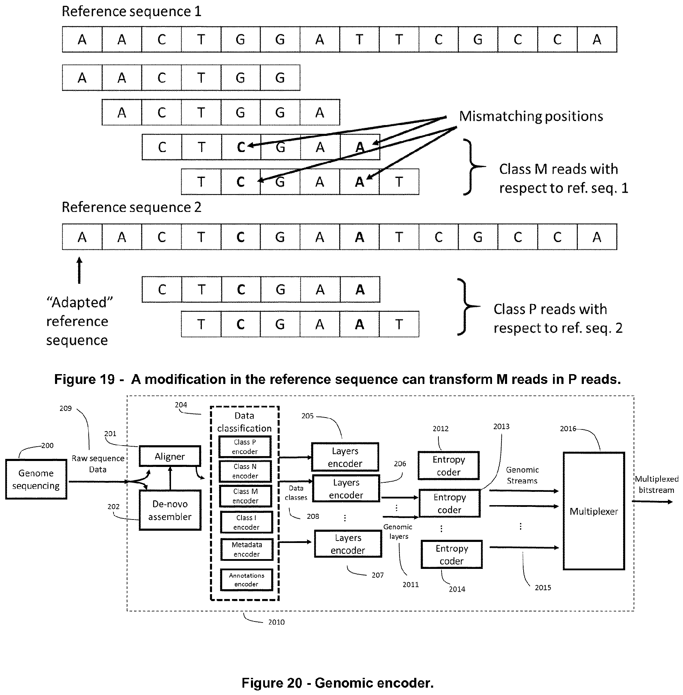

Figure 19 shows how reads containing mismatches (M reads) with respect to reference sequence 1 (RS1) can be transformed into perfectly matching reads (P reads) with respect to reference sequence 2 (RS2) obtained from RS1 by modifying the mismatching positions. This transformation can be expressed as

$$RS2 = A(RS1)$$

If the expression of transformation A which goes from RS1 to RS2 requires less bits of the expression of the mismatches present in the M reads, this encoding method results in a smaller information entropy and therefore better compression.

**Source models, entropy coders and coding modes**

For each layer of the genomic data structure disclosed in this invention different coding algorithms may be adopted according to the specific features of the data or metadata carried by the layer and its statistical properties. The "coding algorithm" has to be intended as the association of a specific "source model" of the descriptor with a specific "entropy coder". The specific "source model" can be specified and selected to obtain the most efficient coding of the data in terms of minimization of the source entropy. The selection of the entropy coder can be driven by coding efficiency considerations and/or probability distribution features and associated implementation issues. Each selection of a specific coding algorithm will be referred to as "coding mode" applied to an entire "layer".

Each "source model" associated to a coding mode is characterized by:

- The definition of the syntax elements emitted by each source (e.g. reads position, reads pairing information, mismatches with respect to a reference sequence etc.)
- The definition of the associated probability model.
- The definition of the associated entropy coder.

**Further advantages**

This classification permits the implementation of efficient coding modes exploiting the lower information source entropy characterizing by modelling the sequences of syntax elements by single separate data sources (e.g. distance, position, etc.).

Another advantage of the invention is the possibility to access only the subset of type of data of interest. For example one of the most important application in genomics consists in finding the differences of a genomic sample with respect to a reference (SNV) or a population (SNP). Today such type of analysis requires the processing of the complete sequence reads whereas by adopting the data representation disclosed by the invention the mismatches are already isolated into one to three data classes only (depending on the interest in considering N codes and indels).

A further advantage is the possibility of performing efficient transcoding from data and metadata compressed with reference to a specific "reference sequence" to another "reference sequence" when a new "reference sequence" is published or when re-mapping is performed on the already mapped data (e.g. using a different mapping algorithm).

Figure 20 shows an encoding apparatus 207 according to the principles of this invention. The encoding apparatus 207 receives as input a raw sequence data 209, for example produced by a genome sequencing apparatus 200. Genome sequencing apparatus 200 are known in the art, like the Illumina HiSeq 2500 or the Thermo-Fisher Ion Torrent devices. The raw sequence data 209 is fed to an aligner unit 201, which prepares the sequences for encoding by aligning the reads to a reference sequence. Alternatively, a de-novo assembler 202 can be used to create a reference sequence from the available reads by looking for overlapping prefixes or suffixes so that longer segments (called "contigs") can be assembled from the reads. After having been processed by a de-novo assembler 202, reads can be mapped on the obtained longer sequence. The aligned sequences are then classified by data classification module 204. The data classes 208 are then fed to layers encoders 205-207. The genomic layers 2011 are then fed to arithmetic encoders 2012-2014 which encode the layers according to the statistical properties of the data or metadata carried by the layer. The result is a genomic stream 2015.

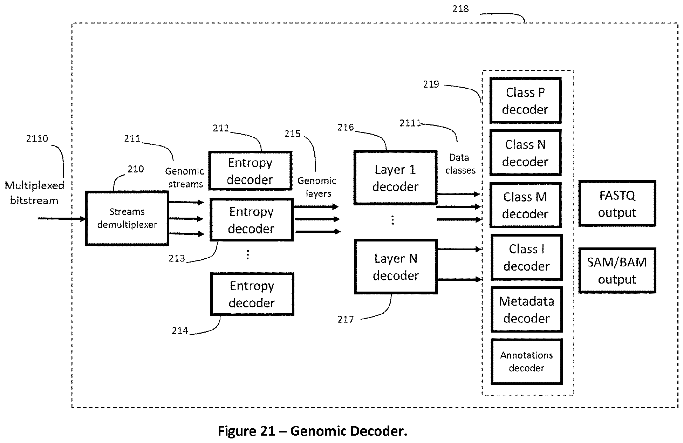

Figure 21 shows a decoding apparatus 218 according to the principles of this disclosure. A decoding apparatus 218 receives a multiplexed genomic bitstream 2110 from a network or a storage element. The multiplexed genomic bitstream 2110 is fed to a demultiplexer 210, to produce separate streams 211 which are then fed to entropy decoders 212-214, to produce genomic layers 215. The extracted genomic layers are fed to layer decoders 216-217 to further decode the layers into classes of data. Class decoders 219 further process the genomic descriptors and merge the results to produce uncompressed reads of sequences, which can then be further stored in the formats known in the art, for instance a text file or zip compressed file, or FASTQ or SAM/BAM files.

Class decoders 219 are able to reconstruct the original genomic sequences by leveraging the information on the original reference sequences carried by one or more genomic streams. In case the reference sequences are not transported by the genomic streams they must be available at the decoding side and accessible by the class decoders.

The inventive techniques herewith disclosed may be implemented in hardware, software, firmware or any combination thereof. When implemented in software, these may be stored on a computer medium and executed by a hardware processing unit. The hardware processing unit may comprise one or more processors, digital signal processors, general purpose microprocessors, application specific integrated circuits or other discrete logic circuitry.

The techniques of this disclosure may be implemented in a variety of devices or apparatuses, including mobile phones, desktop computers, servers, tablets and similar devices.

**Claims**

1. A computer-implemented method for the compression of genome sequence data, said genome sequence data comprising reads of sequences of nucleotides,

said method comprising the steps of:

- aligning said reads to one or more reference sequences thereby creating aligned reads,
- classifying said aligned reads into different classes at least comprising:

- a first class: when said aligned reads match said one or more reference sequences without any error;
- a second class: when said aligned reads match a region in said one or more reference sequences with a number of mismatches constituted by a number of positions in which the sequencing machine was not able to call any base;
- a third class: when said aligned reads match a region in said one or more reference sequences with a number of mismatches constituted by a number of positions in which the sequencing machine was not able to call any base or it called a different base than the one reported in the reference genome.
- a fourth class: when said aligned reads match a region in said one or more reference sequences with a number of mismatches constituted by a number of positions in which the sequencing machine was not able to call any base, or it called a different base than the one reported in the reference genome and by the presence of insertions or deletions or clipped nucleotides;
- a fifth class: when said aligned reads do not find any valid mapping on said one or more reference sequences according to specified alignment constraints,

thereby creating classes of aligned reads;
- encoding said classified and aligned reads as a multiplicity of layers of syntax elements defined by descriptors that univocally represent genome sequence reads, said descriptors comprising for said first class at least the start position on the reference sequence, a flag signaling if the read has to be considered as a reverse complement versus the reference, a distance to the mate pair in case of paired reads, the value of the length in case of the sequencing technology produces variable length reads, said descriptors comprising for said second class at least the descriptors of said first class and a mismatch position for each mismatch, said descriptors comprising for said third class the descriptors of said second class and a mismatch position and a mismatch type for each mismatch, said descriptors comprising for said fourth class the descriptors of said first class and, mismatch type for each mismatch, and the clipped bases,

wherein encoding said classified aligned reads as a multiplicity of layers of syntax elements comprises selecting said syntax elements comprising said descriptors according to said classes of aligned reads,
wherein the encoding of said classified aligned reads as a multiplicity of layers of syntax elements is adapted according to the statistical properties of said selected syntax elements,
wherein the encoding of said classified aligned reads as a multiplicity of layers of syntax elements comprising said descriptors associates a specific source model and a specific entropy coder to each layer, the source model being **characterized by** the definition of the syntax elements emitted by each source, the definition of an associated probability model and the definition of the associated entropy coder, wherein there is decomposition of the sequence read data and metadata into homogeneous layers of said descriptors in order to obtain distinct information sources with reduced information entropy.

2. The method of claim 1, wherein said layers of syntax elements further comprise the position of a variant with respect to the reference sequence, the type of variant, the position of a deletion with respect to the reference sequence, the position of one or more symbols not present in the reference sequence, but present in the aligned reads, the type of insertion at a given position.

3. The method of claim 1, wherein said entropy coder is a context adaptive arithmetic coder.

4. A method for the decompression of a genomic stream compressed according to the method of claim 1, said method comprising the steps of:

- parsing and decoding (212-214) the compressed genomic stream into genomic layers of syntax elements (215),
- decoding said genomic layers into classes of data (216-217),
- expanding said genomic layers into classified reads of sequences of nucleotides,
- selectively decoding using class decoders (219) said classified reads of sequences of nucleotides and merging the result on one or more reference sequences so as to produce uncompressed reads of sequences of nucleotides.

5. A genomic encoder device (2010) for the compression of genome sequence data (209), said genome sequence data (209) comprising reads of sequences of nucleotides, said genomic encoder (2010) comprising:

- an aligner unit (201), configured to align said reads to one or more reference sequences thereby creating aligned reads,
- a data classification unit (204), configured to classify said aligned reads into different classes at least comprising:

- a first class: when said aligned reads match said one or more reference sequence without any error;
- a second class: when said aligned reads match a region in said one or more reference sequences with a number of mismatches constituted by a number of positions in which the sequencing machine was not able to call any base;
- a third class: when said aligned reads match a region in said one or more reference sequences with a number of mismatches constituted by a number of positions in which the sequencing machine was not able to call any base or it called a different base than the one reported in the reference genome.
- a fourth class: when said aligned reads match a region in said one or more reference sequences with a number of mismatches constituted by a number of positions in which the sequencing machine was not able to call any base, or it called a different base than the one reported in the reference genome and by the presence of insertions or deletions or clipped nucleotides;
- a fifth class: when said aligned reads do not find any valid mapping on said one or more reference sequences according to specified alignment constraints,

thereby creating classes of aligned reads;
- one or more layers encoding units (205-207), configured to encode said classified aligned reads as layers of syntax elements by selecting said syntax elements according to said classes of aligned reads, wherein said descriptors comprise for said first class at least the start position on the reference sequence, a flag signaling if the read has to be considered as a reverse complement versus the reference, a distance to the mate pair in case of paired reads, the value of the length in case of the sequencing technology produces variable length reads, said descriptors comprising for said second class at least the descriptors of said first class and a mismatch position for each mismatch, said descriptors comprising for said third class the descriptors of said second class and a mismatch position and a mismatch type for each mismatch, said descriptors comprising for said fourth class the descriptors of said first class and a mismatch type for each mismatch and the clipped bases;
- entropy coder units (2012-2014) for entropy coding said layers of syntax elements,
wherein the encoding of said classified aligned reads as a multiplicity of layers of syntax elements is adapted according to the statistical properties of the data carried by the layer,
wherein the encoding of said classified aligned reads as a multiplicity of layers of syntax elements comprising said descriptors associates a specific source model and a specific entropy coder to each layer, the source model being **characterized by** the definition of the syntax elements emitted by each source, the definition of an associated probability model and the definition of the associated entropy coder,
wherein there is decomposition of the sequence read data and metadata into homogeneous layers of said descriptors in order to obtain distinct information sources with reduced information entropy.

6. A genomic decoder device (218) for the decompression of a genomic stream (211) compressed by the genomic encoder of claim 5, said genomic decoder (218) comprising:

- parsing and decoding means (210, 212-214) configured to parse said compressed genomic stream into genomic layers of syntax elements (215),
- one or more layer decoders (216-217), configured to decode the genomic layers into classes of data and further configured to process said genomic layers into classified reads of sequences of nucleotides (2111),
- genomic data classes decoders (213) configured to selectively decode said classified reads of sequences of nucleotides and configured to merge the result on one or more reference sequences so as to produce uncompressed reads of sequences of nucleotides.

7. The genomic decoder device of claim 6, wherein the one or more reference sequences are stored in the compressed genomic stream (211).

8. The genomic decoder device of claim 6, wherein the one or more reference sequences are provided to the decoder via an out of band mechanism.

9. The genomic decoder device of claim 6, wherein the one or more reference sequences are built at the decoder.

10. A computer-readable medium comprising instructions that when executed cause at least one processor to perform the method of any one of claims 1 to 4.

**Patentansprüche**

1. Computerimplementiertes Verfahren für die Kompression von Genomsequenz-Daten, wobei die Genomsequenz-Daten Reads von Sequenzen von Nukleotiden umfassen, wobei das Verfahren die Schritte umfasst:

   - Alignieren der Reads mit einer oder mehreren Referenzsequenzen, wodurch alignierte Reads geschaffen werden,
   - Klassifizieren der alignierten Reads in Klassen, die zumindest umfassen:
   - eine erste Klasse: wenn die alignierten Reads mit der einen oder den mehreren Referenzsequenzen ohne jeglichen Fehler übereinstimmen;
   - eine zweite Klasse: wenn die alignierten Reads mit einer Region in der einen oder den mehreren Referenzsequenzen mit einer Anzahl von Übereinstimmungsfehlern übereinstimmen, die durch eine Anzahl von Positionen gebildet werden, in welchen die Sequenziermaschine keine Base nennen konnte,
   - eine dritte Klasse: wenn die alignierten Reads mit einer Region in der einen oder den mehreren Referenzsequenzen mit einer Anzahl von Übereinstimmungsfehlern übereinstimmen, die durch eine Anzahl von Positionen gebildet werden, in welchen die Sequenziermaschine keine Base nennen konnte, oder sie eine andere Base genannt hat als jene, die in dem Referenzgenom berichtet wurde;
   - eine vierte Klasse: wenn die alignierten Reads mit einer Region in der einen oder den mehreren Referenzsequenzen mit einer Anzahl von Übereinstimmungsfehlern übereinstimmen, die durch eine Anzahl von Positionen, in welchen die Sequenziermaschine keine Base nennen konnte, oder sie eine andere Base genannt hat als jene, die in dem Referenzgenom berichtet wurde, und durch das Vorliegen von Insertionen oder Deletionen oder abgeschnittenen Nukleotiden gebildet werden;
   - eine fünfte Klasse: wenn die alignierten Reads in Übereinstimmung mit den angegebenen Alignment-Beschränkungen kein gültiges Mapping auf der einen oder den mehreren Referenzsequenzen finden,

   wodurch Klassen von alignierten Reads geschaffen werden;

   Kodieren der klassifizierten und alignierten Reads als eine Vielzahl von Schichten von Syntax-Elementen, die durch Deskriptoren definiert werden, die eindeutig Genomsequenz-Reads repräsentieren, wobei die Deskriptoren für die erste Klasse zumindest die Startposition an der Referenzsequenz, ein Flag, das signalisiert, wenn das Read als ein reverses Komplement in Bezug auf die Referenz angesehen werden muss, einen Abstand zu dem übereinstimmenden Paar im Fall von gepaarten Reads und den Wert der Länge in dem Fall, dass die Sequenzierungstechnologie Reads variabler Länge produziert, umfassen; wobei die Deskriptoren für die zweite Klasse zumindest die Deskriptoren der ersten Klasse und eine Übereinstimmungsfehler-Position für jeden Übereinstimmungsfehler umfassen, wobei die Deskriptoren für die dritte Klasse die Deskriptoren der zweiten Klasse und eine Übereinstimmungsfehler-Position und einen Übereinstimmungsfehler-Typ für jeden Übereinstimmungsfehler umfassen, wobei die Deskriptoren für die vierte Klasse die Deskriptoren der ersten Klasse und (einen) Übereinstimmungsfehler-Typ für jeden Übereinstimmungsfehler und die abgeschnittenen Basen umfassen,
   wobei das Kodieren der klassifizierten alignierten Reads als eine Vielzahl von Schichten von Syntaxelementen das Auswählen der Syntaxelemente umfasst, welche die Deskriptoren in Übereinstimmung mit den Klassen von alignierten Reads umfassen,
   wobei das Kodieren der klassifizierten alignierten Reads als eine Vielzahl von Schichten von Syntaxelementen in Übereinstimmung mit den statistischen Eigenschaften der ausgewählten Syntaxelemente adaptiert wird,
   wobei das Kodieren der klassifizierten alignierten Reads als eine Vielzahl von Schichten von Syntaxelementen umfassend die Deskriptoren jeder Schicht ein spezifisches Quellenmodell und einen spezifischen Entropie-Kodierer zuordnet, wobei das Quellenmodell durch die Definition der von jeder Quelle ausgegebenen Syntaxelemente, die Definition eines zugeordneten Wahrscheinlichkeitsmodells und die Definition des zugeordneten Entropie-Kodierers gekennzeichnet ist, wobei eine Dekomposition der Sequenz-Read-Daten und Metadaten in homogene Schichten der Deskriptoren erfolgt, um distinkte Informationsquellen mit reduzierter Informationsentropie zu erhalten.

**2.** Verfahren nach Anspruch 1, wobei die Schichten von Syntaxelementen ferner die Position einer Variante in Bezug auf die Referenzsequenz, den Typ der Variante, die Position einer Deletion in Bezug auf die Referenzsequenz, die Position eines oder mehrerer Symbole, die in der Referenzsequenz nicht vorhanden sind, in den alignierten Reads jedoch vorhanden sind, und den Typ der Insertion an einer gegebenen Position umfassen.

**3.** Verfahren nach Anspruch 2, wobei der Entropie-Kodierer ein kontext-adaptiver arithmetischer Kodierer ist.

**4.** Verfahren für die Dekompression eines genomischen Datenstroms, der gemäß dem Verfahren nach Anspruch 1 komprimiert wurde, wobei das Verfahren die folgenden Schritte umfasst:

- Parsen und Dekodieren (212-214) des komprimierten genomischen Stroms in genomische Schichten von Syntaxelementen (215),
- Dekodieren der genomischen Schichten in Klassen von Daten (216-217),
- Expandieren der genomischen Schichten zu klassifizierten Reads von Sequenzen von Nukleotiden,
- selektives Dekodieren, unter Verwendung von Klassen-Dekodern (2319), der klassifizierten Reads von Sequenzen von Nukleotiden und Zusammenfügen des Ergebnisses an einer oder mehreren Referenzsequenzen, um unkomprimierte Reads von Sequenzen von Nukleotiden zu produzieren.

**5.** Genomische Kodiervorrichtung (2010) für die Kompression von Genomsequenz-Daten (209), wobei die Genomsequenz-Daten (209) Reads von Sequenzen von Nukleotiden umfassen, wobei der genomische Kodierer (2010) umfasst:

- eine Aligner-Einheit (201), die dazu ausgestaltet ist, die Reads mit einer oder mehreren Referenzsequenzen zu alignieren, wodurch alignierte Reads geschaffen werden;
- eine Daten-Klassifizierungseinheit (204), die dazu ausgestaltet ist, die alignierten Reads in unterschiedliche Klassen zu klassifizieren, die zumindest umfassen:
- eine erste Klasse: wenn die alignierten Reads mit der einen oder den mehreren Referenzsequenzen ohne jeglichen Fehler übereinstimmen;
- eine zweite Klasse: wenn die alignierten Reads mit einer Region in der einen oder den mehreren Referenzsequenzen mit einer Anzahl von Übereinstimmungsfehlern übereinstimmen, die durch eine Anzahl von Positionen gebildet werden, in welchen die Sequenziermaschine keine Base nennen konnte,
- eine dritte Klasse: wenn die alignierten Reads mit einer Region in der einen oder den mehreren Referenzsequenzen mit einer Anzahl von Übereinstimmungsfehlern übereinstimmen, die durch eine Anzahl von Positionen gebildet werden, in welchen die Sequenziermaschine keine Base nennen konnte, oder sie eine andere Base genannt hat als jene, die in dem Referenzgenom berichtet wurde;
- eine vierte Klasse: wenn die alignierten Reads mit einer Region in der einen oder den mehreren Referenzsequenzen mit einer Anzahl von Übereinstimmungsfehlern übereinstimmen, die durch eine Anzahl von Positionen, in welchen die Sequenziermaschine keine Base nennen konnte, oder sie eine andere Base genannt hat als jene, die in dem Referenzgenom berichtet wurde, und durch das Vorliegen von Insertionen oder Deletionen oder abgeschnittenen Nukleotiden gebildet werden;
- eine fünfte Klasse: wenn die alignierten Reads mit einer Region in der einen oder den mehreren Referenzsequenzen mit einer Anzahl von Übereinstimmungsfehlern übereinstimmen, die durch eine Anzahl von Positionen, in welchen die Sequenziermaschine keine Base nennen konnte, oder sie eine andere Base genannt hat als jene, die in dem Referenzgenom berichtet wurde, und durch das Vorliegen von Insertionen oder Deletionen oder abgeschnittenen Nukleotiden gebildet werden;
wodurch Klassen von alignierten Reads geschaffen werden;

- eine oder mehrere Schichten-Kodiereinheiten (205-207), die dazu ausgestaltet sind, die klassifizierten alignierten Reads als Schichten von Syntaxelementen zu kodieren, indem sie Syntaxelemente in Übereinstimmung mit den Klassen von alignierten Reads auswählen, wobei die Deskriptoren für die erste Klasse zumindest die Startposition an der Referenzsequenz, ein Flag, das signalisiert, wenn das Read als ein reverses Komplement in Bezug auf die Referenz angesehen werden muss, einen Abstand zu dem übereinstimmenden Paar im Fall von gepaarten Reads und den Wert der Länge in dem Fall, dass die Sequenzierungstechnologie Reads variabler Länge produziert, umfassen; wobei die Deskriptoren für die zweite Klasse zumindest die Deskriptoren der ersten Klasse und eine Übereinstimmungsfehler-Position für jeden Übereinstimmungsfehler umfassen, wobei die Deskriptoren für die dritte Klasse die Deskriptoren der zweiten Klasse und eine Übereinstimmungsfehler-Position und einen Übereinstimmungsfehler-Typ für jeden Übereinstimmungsfehler umfassen, wobei die Deskriptoren für die vierte Klasse die Deskriptoren der ersten Klasse und (einen) Übereinstimmungsfehler-Typ für jeden Übereinstimmungsfehler und die abgeschnittenen Basen umfassen;
- Entropie-Kodierer-Einheiten (2012-2014) zum Entropie-Kodieren der Schichten von Syntaxelementen,

wobei das Kodieren der klassifizierten alignierten Reads als eine Vielzahl von Schichten von Syntaxelementen in Übereinstimmung mit den statistischen Eigenschaften der von der Schicht getragenen Daten adaptiert wird, wobei das Kodieren der klassifizierten alignierten Reads als eine Vielzahl von Schichten von Syntaxelementen umfassend die Deskriptoren jeder Schicht ein spezifisches Quellenmodell und einen spezifischen Entropie-Kodierer zuordnet, wobei das Quellenmodell durch die Definition der von jeder Quelle ausgegebenen Syntaxelemente, die Definition eines zugeordneten Wahrscheinlichkeitsmodells und die Definition des zugeordneten Entropie-Kodierers gekennzeichnet ist,
wobei eine Dekomposition der Sequenz-Read-Daten und Metadaten in homogene Schichten der Deskriptoren erfolgt, um distinkte Informationsquellen mit reduzierter Informationsentropie zu erhalten.

6. Genomische Dekodierer-Vorrichtung (218) für die Dekompression eines genomischen Stroms (211), der durch den genomischen Kodierer nach Anspruch 5 komprimiert wurde, wobei der genomische Dekodierer (218) umfasst:

- Parser- und Dekodiermittel (210, 212-214), die zum Parsen des komprimierten genomischen Stroms in genomische Schichten von Syntaxelementen (215) ausgestaltet ist,
- einen oder mehrere Schicht-Dekoder (216-217), die dazu ausgestaltet sind, die genomischen Schichten in Klassen von Daten zu dekodieren, und ferner dazu ausgestaltet sind, die genomischen Schichten zu klassifizierten Reads von Sequenzen von Nukleotiden (2111) zu verarbeiten,
- genomische Datenklassen-Dekoder (213), die dazu ausgestaltet sind, die klassifizierten Reads von Sequenzen von Nukleotiden zu dekodieren, und dazu ausgestaltet sind, das Ergebnis an einer oder mehreren Referenzsequenzen zusammenzufügen, um unkomprimierte Reads von Sequenzen von Nukleotiden zu produzieren.

7. Genomische Dekodierer-Vorrichtung nach Anspruch 6, wobei die eine oder die mehreren Referenzsequenzen in dem komprimierten genomischen Strom (211) gespeichert sind.

8. Genomische Dekodierer-Vorrichtung nach Anspruch 6, wobei die eine oder die mehreren Referenzsequenzen dem Dekodierer durch einen Out-of-the-Band-Mechanismus bereitgestellt werden.

9. Genomische Dekodierer-Vorrichtung nach Anspruch 6, wobei die eine oder die mehreren Referenzsequenzen in dem Dekodierer aufgebaut werden.

10. Computerlesbares Medium umfassend Anweisungen, die, wenn sie ausgeführt werden, zumindest einen Prozessor veranlassen, das Verfahren nach einem der Ansprüche 1 bis 4 durchzuführen.

**Revendications**

1. Procédé mis en oeuvre par ordinateur pour la compression de données de séquences génomiques, lesdites données de séquences génomiques comprenant des lectures de séquences de nucléotides,
ledit procédé comprenant les étapes de:

- alignement desdites lectures sur une ou plusieurs séquences de référence créant ainsi des lectures alignées,
- classement desdites lectures alignées en différentes classes comprenant au moins:

- une première classe: lorsque lesdites lectures alignées correspondent auxdites une ou plusieurs séquences de référence sans aucune erreur;
- une seconde classe: lorsque lesdites lectures alignées correspondent à une région dans lesdites une ou plusieurs séquences de référence avec un certain nombre de mésappariements constitués par un certain nombre de positions dans lesquelles la machine de séquençage n'a pu appeler aucune base,
- une troisième classe: lorsque lesdites lectures alignées correspondent à une région dans lesdites une ou plusieurs séquences de référence avec un certain nombre de mésappariements constitués par un certain nombre de positions dans lesquelles la machine de séquençage n'a pu appeler aucune base ou a appelé une autre base que celle rapportée dans le génome de référence.
- une quatrième classe: lorsque lesdites lectures alignées correspondent à une région de lesdites une ou plusieurs séquences de référence avec un certain nombre de mésappariements constitués par un certain nombre de positions dans lesquelles la machine de séquençage n'a pu appeler aucune base, ou a appelé une autre base que celle rapportée dans le génome de référence et par la présence d'insertions ou de délétions ou de nucléotides coupés;

- une cinquième classe: lorsque lesdites lectures alignées ne trouvent aucune cartographie valide sur lesdites une ou plusieurs séquences de référence selon des contraintes d'alignement spécifiées,

créant ainsi des classes de lectures alignées,

- codage desdites lectures classées et alignées sous la forme d'une multiplicité de couches d'éléments de syntaxe définis par des descripteurs qui représentent de manière univoque des lectures de séquences génomiques, lesdits descripteurs comprenant pour ladite première classe au moins la position de départ sur la séquence de référence, un drapeau signalant si la lecture doit être considérée comme un complément inverse par rapport à la référence, une distance à la paire d'accouplement dans le cas de lectures appariées, la valeur de la longueur dans le cas où la technologie de séquençage produit des lectures de longueur variable, lesdits descripteurs comprenant pour ladite seconde classe au moins les descripteurs de ladite première classe et une position de mésappariement pour chaque mésappariement, lesdits descripteurs comprenant pour ladite troisième classe les descripteurs de ladite seconde classe et une position de mésappariement et un type de mésappariement pour chaque mésappariement, lesdits descripteurs comprenant pour ladite quatrième classe les descripteurs de ladite première classe et le type de mésappariement pour chaque mésappariement, et les bases coupées,

dans lequel le codage desdites lectures alignées classées sous la forme d'une multiplicité de couches d'éléments de syntaxe comprend la sélection desdits éléments de syntaxe comprenant lesdits descripteurs selon lesdites classes de lectures alignées,

dans lequel le codage desdites lectures alignées classées sous la forme d'une multiplicité de couches d'éléments de syntaxe est adapté selon les propriétés statistiques desdits éléments de syntaxe sélectionnés,

dans lequel le codage desdites lectures alignées classées sous la forme d'une multiplicité de couches d'éléments de syntaxe comprenant lesdits descripteurs associe un modèle source spécifique et un codeur d'entropie spécifique à chaque couche, le modèle source étant **caractérisé par** la définition des éléments de syntaxe émis par chaque source, la définition d'un modèle de probabilité associé et la définition du codeur d'entropie associé,

dans lequel il y a décomposition des données et métadonnées de lectures de séquences en couches homogènes desdits descripteurs afin d'obtenir des sources d'information distinctes avec une entropie d'information réduite.

2. Procédé selon la revendication 1, dans lequel lesdites couches d'éléments de syntaxe comprennent en outre la position d'un variant par rapport à la séquence de référence, le type de variant, la position d'une délétion par rapport à la séquence de référence, la position d'un ou plusieurs symboles non présents dans la séquence de référence, mais présents dans les lectures alignées, le type d'insertion à une position donnée.

3. Procédé selon la revendication 1, dans lequel ledit codeur d'entropie est un codeur arithmétique adaptatif au contexte.

4. Procédé de décompression d'un flux génomique compressé selon le procédé selon la revendication 1, ledit procédé comprenant les étapes de:

- analyse et décodage (212-214) du flux génomique compressé en couches génomiques d'éléments de syntaxe (215),
- décodage desdites couches génomiques en classes de données (216-217),
- expansion desdites couches génomiques en lectures de séquences de nucléotides classées,
- décodage sélectif à l'aide de décodeurs de classe (219) desdites lectures de séquences de nucléotides classées et fusion du résultat sur une ou plusieurs séquences de référence afin de produire des lectures de séquences de nucléotides non compressées.

5. Dispositif codeur génomique (2010) pour la compression de données de séquences génomiques (209), lesdites données de séquences génomiques (209) comprenant des lectures de séquences de nucléotides, ledit codeur génomique (2010) comprenant:

- une unité d'aligneur (201), configurée pour aligner lesdites lectures sur une ou plusieurs séquences de référence créant ainsi des lectures alignées,
- une unité de classification de données (204), configurée pour classer lesdites lectures alignées en différentes classes comprenant au moins:

- une première classe: lorsque lesdites lectures alignées correspondent auxdites une ou plusieurs séquences de référence sans aucune erreur;
- une seconde classe: lorsque lesdites lectures alignées correspondent à une région dans lesdites une ou plusieurs séquences de référence avec un certain nombre de mésappariements constitués par un certain nombre de positions dans lesquelles la machine de séquençage n'a pu appeler aucune base,
- une troisième classe: lorsque lesdites lectures alignées correspondent à une région dans lesdites une ou plusieurs séquences de référence avec un certain nombre de mésappariements constitués par un certain nombre de positions dans lesquelles la machine de séquençage n'a pu appeler aucune base ou a appelé une autre base que celle rapportée dans le génome de référence.
- une quatrième classe: lorsque lesdites lectures alignées correspondent à une région dans lesdites une ou plusieurs séquences de référence avec un certain nombre de mésappariements constitués par un certain nombre de positions dans lesquelles la machine de séquençage n'a pu appeler aucune base, ou a appelé une autre base que celle rapportée dans le génome de référence et par la présence d'insertions ou de délétions ou de nucléotides coupés;
- une cinquième classe: lorsque lesdites lectures alignées ne trouvent aucune cartographie valide sur lesdites une ou plusieurs séquences de référence selon des contraintes d'alignement spécifiées, créant ainsi des classes de lectures alignées,

- une ou plusieurs unités codant des couches (205-207), configurées pour coder lesdites lectures alignées classées sous la forme de couches d'éléments de syntaxe en sélectionnant lesdits éléments de syntaxe selon lesdites classes de lectures alignés, dans lequel lesdits descripteurs comprennent pour ladite première classe au moins la position de départ sur la séquence de référence, un drapeau signalant si la lecture doit être considérée comme un complément inverse par rapport à la référence, une distance à la paire d'accouplement en cas de lectures appariées, la valeur de la longueur dans le cas où la technologie de séquençage produit des lectures de longueur variable, lesdits descripteurs comprenant pour ladite seconde classe au moins les descripteurs de ladite première classe et une position de mésappariement pour chaque mésappariement, lesdits descripteurs comprenant pour ladite troisième classe les descripteurs de ladite seconde classe et une position de mésappariement et un type de mésappariement pour chaque mésappariement, lesdits descripteurs comprenant pour ladite quatrième classe les descripteurs de ladite première classe et un type de mésappariement pour chaque mésappariement et les bases coupées;
- des unités de codeur d'entropie (2012-2014) pour le codage d'entropie desdites couches d'éléments de syntaxe,
dans lequel le codage desdites lectures alignées classées sous la forme d'une multiplicité de couches d'éléments de syntaxe est adapté selon les propriétés statistiques des données portées par la couche,
dans lequel le codage desdites lectures alignées classées sous la forme d'une multiplicité de couches d'éléments de syntaxe comprenant lesdits descripteurs associe un modèle source spécifique et un codeur d'entropie spécifique à chaque couche, le modèle source étant **caractérisé par** la définition des éléments de syntaxe émis par chaque source, la définition d'un modèle de probabilité associé et la définition du codeur d'entropie associé, dans lequel il y a décomposition des données et métadonnées de lectures de séquences en couches homogènes desdits descripteurs afin d'obtenir des sources d'information distinctes avec une entropie d'information réduite.

6. Dispositif décodeur génomique (218) pour la décompression d'un flux génomique (211) compressé par le codeur génomique selon la revendication 5, ledit décodeur génomique (218) comprenant:

- des moyens d'analyse et de décodage (210, 212-214) configurés pour analyser ledit flux génomique compressé en couches génomiques d'éléments de syntaxe (215),
- un ou plusieurs décodeurs de couches (216-217), configurés pour décoder les couches génomiques en classes de données et configurés en outre pour traiter lesdites couches génomiques en lectures de séquences de nucléotides classées (2111),
- des décodeurs de classes de données génomiques (213) configurés pour décoder sélectivement lesdites lectures de séquences de nucléotides classées et configurés pour fusionner le résultat sur une ou plusieurs séquences de référence afin de produire des lectures de séquences de nucléotides non compressées.

7. Dispositif décodeur génomique selon la revendication 6, dans lequel les une ou plusieurs séquences de référence sont stockées dans le flux génomique compressé (211).

8. Dispositif décodeur génomique selon la revendication 6, dans lequel les une ou plusieurs séquences de référence sont fournies au décodeur via un mécanisme hors bande.

9. Dispositif décodeur génomique selon la revendication 6, dans lequel les une ou plusieurs séquences de référence sont construites au niveau du décodeur.

10. Support lisible par ordinateur comprenant des instructions qui, lorsqu'elles sont exécutées, amènent au moins un processeur à mettre en oeuvre le procédé selon l'une quelconque des revendications 1 à 4.

Pair 1 = Read 1 + Read 2
Pair 2 = Read 3 + Read 4
Pair 3 = Read 5 + Read 6

| | Read 1 | | Read 3 | | Read 2 | | Read 4 | | Read 6 | | Read 5 |

*Reference sequence*

p0 = 0    p1 = 10000    p3 = 10180    p2 = 10320   p4 = 10450   p6 = 10620   p5 = 10850

*pos* Layer

| 10000 | 180 | 670 |

p3 − p1 = 180
p5 − p3 = 670

**Figure 1 - How the position of the first read of three read pairs mapped are encoded in the pos layer.**

strand 1

| A | A | C | T | G | G | A | T | T | C | G | A | T | A |

| A | C | T | G |
read 1

strand 2

| T | T | G | A | C | C | T | A | A | G | C | T | A | T |

| A | G | C | T |
read 2

**Figure 2 - In this reads pair read 1 comes from strand 1 and read 2 from strand 2.**

strand 1

| A | A | C | T | G | G | A | T | T | C | G | A | T | A |

reverse complement of read 2

**Figure 3 - The reverse complement of read 2 will be encoded if strand 1 is used as reference.**

23

**Figure 4 - The four possible combinations of reads composing a reads pair and the respective encoding in the rcomp layer.**

*EXAMPLE*
*Constant reads length = 100*

$$p2 - p1 - 100 = 220$$
$$p4 - p3 - 100 = 170$$
$$p6 - p5 - 100 = -330$$

**Figure 5 - Calculation of pairing distance for three read pairs.**

EXAMPLE
Most Probable Position Pairing Distance (MPPPD) = 2

pairing error = -1

MPPPD    pairing error = 2

MPPPD

| R1 | R4 | R3 | R5 | R2 | R7 | R8 | R6 | R9 | R11 | R10 | R12 |

Reference sequence

pair

| 2 | -3 | 2 | -1 | 0 | 0 | | | | | Pairing errors vector |

Optionally the pair layer can be differentially encoded into pair'

pair'

| 2 | -5 | 5 | -3 | 1 | 0 | | | | | Pairing errors vector |

**Figure 6 - Calculation of pairing errors.**

Pair 1 = Read 1 + Read 2
Pair 2 = Read 3 + Read 4
Pair 3 = Read 5 + Read 6

EXAMPLE
Constant reads length = 100

Read 1        Read 3        Read 2                    Read 6        Read 5

Reference sequence 1

Read 4

Reference sequence 4

p0 = 0    p1 = 10000    p3 = 10180    p2 = 10320    p4 = 10450    p6 = 10620    p5 = 10850

**Pairing distances**

| 220 | 0xffffff | 4 | 170 | -330 |

p2 – p1 – 100 = 220
p4 – p3 – 100 = 170
p6 – p5 – 100 = -330

reserved value (flag)

reference sequence id if different from current reference

**Figure 7 – When a read is mapped on a difference reference than its mate (read 4), additional descriptors are added to the pairing distance. One is a signaling flag, the second is a reference identifier and then the pairing distance.**

| Ref. | A | A | C | T | G | G | A | T | T | C | G | A | T | A |
|------|---|---|---|---|---|---|---|---|---|---|---|---|---|---|

Read 1: A N T N

Read 2: N G A N

| nmis layer | | | 1 | 3 | 4 | 7 | S | | | | | |
|---|---|---|---|---|---|---|---|---|---|---|---|---|

| nmis layer (differential enc.) | 1 | 2 | 1 | 3 | S | | | | | |
|---|---|---|---|---|---|---|---|---|---|---|

**Figure 8 - Calculation of N mismatches in a nmis layer.**

| Ref. | A | A | C | T | G | G | A | T | T | C | G | A | T | A |
|------|---|---|---|---|---|---|---|---|---|---|---|---|---|---|

Read 1: A T T N

Read 2: T G A N

**Figure 9 - Substitutions in a mapped reads pair.**

| Ref. | A | A | C | T | G | G | A | T | T | C | G | A | T | A |
|------|---|---|---|---|---|---|---|---|---|---|---|---|---|---|

Read 1: A C G N

Read 2: C N T T

| Snpp layer | | | 2 | 3 | 5 | 6 | S | | | | | |
|---|---|---|---|---|---|---|---|---|---|---|---|---|---|

| Snpp layer (diff. enc.) | 2 | 1 | 2 | 1 | S | | | | | |
|---|---|---|---|---|---|---|---|---|---|---|

**Figure 10- Calculation of substitutions position either as absolute or differential values.**

# snpt Layer (without IUPAC codes)

A substitution type is calculated as index of a substitutions vector composed by all the possible symbols. For example:

S = [A, C, G, T, N, Z] *where Z = deletion*

Index direction
- ENCODING is from right to left
- DECODING from left to right

| Ref | Read | Encoded Symb. |
|-----|------|---------------|
| A | del. | idx(A,Z) = 5 |
| C | del. | idx(C,Z) = 4 |
| G | del. | idx(G,Z) = 3 |
| T | del. | idx(T,Z) = 2 |

| Ref | Read | Encoded Symb. |
|-----|------|---------------|
| N | A | idx(N,A) = 2 |
| N | C | idx(N,C) = 3 |
| N | G | idx(N,G) = 4 |
| N | T | idx(N,T) = 5 |

| Ref | Read | Encoded Symb. |
|-----|------|---------------|
| A | C | idx(A,C) = 1 |
| A | G | idx(A,G) = 2 |
| A | T | idx(A,T) = 3 |
| A | N | idx(A,N) = 4 |
| C | A | idx(C,A) = 5 |
| C | G | idx(C,G) = 1 |
| C | T | idx(C,T) = 2 |
| C | N | idx(C,N) = 3 |
| G | A | idx(G,A) = 4 |
| G | C | idx(G,C) = 5 |
| G | T | idx(G,T) = 1 |
| G | N | idx(G,N) = 2 |
| T | A | idx(T,A) = 3 |
| T | C | idx(T,C) = 4 |
| T | G | idx(T,G) = 5 |
| T | N | idx(T,N) = 1 |

**Figure 11 - Calculations of symbols encoding substitutions.**

Ref.: A A C T G G A T T C G A T A

Read 1: A C G N

Read 2: C N T T

| | | | | | |
|---|---|---|---|---|---|
| Snpp layer (diff. enc.) | 2 | 1 | 2 | 1 | S |
| Snpt layer | 1 | 4 | 4 | 3 | S |
| Snpt layer (diff. enc.) | 1 | 3 | 0 | -1 | S |

**Figure 12 - Encoding of substitutions into the snpt layer.**

# snpt Layer (with IUPAC codes)

A substitution type is calculated as index in a substitutions vector composed by all the possible symbols. For example:

S = [A, C, G, T, N, Z, M, R, W, S, Y, K, V, H, D, B]

Index direction
- ENCODING is from right to left
- DECODING from left to right

| Ref | Read | Encoded Symb. |
|-----|------|---------------|
| D | M | idx(D,M) = 8 |
| A | Y | idx(A,Y) = 10 |
| A | T | idx(A,T) = 3 |
| A | N | idx(A,N) = 4 |
| C | R | idx(C,R) = 6 |
| C | G | idx(C,G) = 1 |
| C | T | idx(C,T) = 2 |
| C | W | idx(C,W) = 7 |
| G | H | idx(G,H) = 11 |
| G | C | idx(G,C) = 15 |
| G | B | idx(G,B) = 13 |
| G | N | idx(G,N) = 2 |
| T | A | idx(T,A) = 13 |
| T | M | idx(T,M) = 4 |
| T | K | idx(T,K) = 8 |
| T | V | idx(T,V) = 9 |

| Ref | Read | Encoded Symb. |
|-----|------|---------------|
| N | M | idx(N,M) = 2 |
| N | W | idx(N,W) = 4 |
| N | S | idx(N,S) = 5 |
| N | B | idx(N,B) = 11 |

**Figure 13 – Substitution codes when IUPAC ambiguity codes are used.**

S = [A, C, G, T, N, Z, M, R, W, S, Y, K, V, H, D, B]

Direction
- ENCODING is from right to left
- DECODING from left to right

| Ref. | A | A | C | T | G | G | A | T | T | C | G | A | T | A |
|------|---|---|---|---|---|---|---|---|---|---|---|---|---|---|
|  |  | A | C | W | N |  |  |  |  | C | K |  | T |  |

Read 1 ................ Read 2

| Snpp layer (diff. enc.) | 2 | 1 | 2 | 1 | S |  |  |  |  |  |
|-------------------------|---|---|---|---|---|---|---|---|---|---|

| Snpt layer | 5 | 4 | 4 | 9 | S |  |  |  |  |  |
|------------|---|---|---|---|---|---|---|---|---|---|

| Snpt layer (diff. enc.) | 1 | -1 | 0 | 5 | S |  |  |  |  |  |
|-------------------------|---|----|---|---|---|---|---|---|---|---|

**Figure 14 - snpt layer encoding when IUPAC codes are used.**

# indt Layer (without IUPAC codes)

S = [A, C, G, T, N, Z]

Direction

- ENCODING is from right to left
- DECODING from left to right

| Insert | Encoded Sym. |
|--------|--------------|
| A | 6 |
| C | 7 |
| G | 8 |
| T | 9 |
| N | 10 |

Ref.

| A | A | C | T | G | G | A | T | T | C | G | | T | C |

| A | C | | T | G | | | | | C | N | T | T |

Read 1                                                    Read 2

indp layer (diff. enc.)

| 1 | 1 | 4 | 1 | S | | | | | |

indt layer

| 5 | 2 | 4 | 9 | S | | | | | |

indt layer (diff. enc.)

| 5 | -3 | 2 | 5 | S | | | | | |

**Figure 15 - Encoding substitutions, inserts and deletions in a reads pair of class I.**

# indt Layer (with IUPAC codes)

S = [A, C, G, T, N, Z, M, R, W, S, Y, K, V, H, D, B]
Direction
- ENCODING is from right to left
- DECODING from left to right

| Insert | Encoded Sym. |
|--------|--------------|
| A | 16 |
| C | 17 |
| G | 18 |
| T | 19 |
| N | 20 |

Ref.

| A | A | C | T | G | G | A | T | T | C | G | | T | C |

| A | C | | T | G |

Read 1

| C | R | T | W |

Read 2

snpp layer (diff. enc.)

| 1 | 1 | 4 | 1 | 1 | S | | | | |

indt layer

| 15 | 4 | 5 | 19 | 5 | S | | | | |

indt layer (diff. enc.)

| 15 | -11 | 1 | 18 | -14 | S | | | | |

**Figure 16 - Encoding of mismatches and indels in case of IUPAC ambiguity codes.**

# Source model 2 (without IUPAC codes)

One position layer per substitution type, one per deletions and one per insertion type

Ref. | A | A | C | T | G | G | A | T | T | C | G | | T | C

| A | C | | T | T | | | | C | N | T | T

Read 1　　　　　　　　　　　　Read 2

A | 0 | 0

C | 2 | 0 | 6 | 0 — First mismatch to be decoded in this pair. The first layer to be decoded shall be flagged in the access unit header

G | 0 | 2 | 0

T | 5 | 0 | 5 | 9 | 0 — Flag signalling the end of the second read pair. It follows the highest decoded value for the second read

N | 7 | 0

del | 3 | 0 | 3 | 0

insA | 0 | 7 | 0 — Value indicating the absence of mismatches of this type for the encoded read pair

insC | 0 | 0

insG | 0 | 0

insT | 8 | 0 — Flag signalling the end of the first read pair. It follows the highest decoded value for the first read

| A | G | | G | T | Read 3 　　Read 4 | C | A | T | T

Ref. | A | A | C | T | G | G | A | T | T | C | G | | T | C

**Figure 17 - Each layer contains the position of the mismatches or inserts of a single type.**

# Source model 2 (without IUPAC codes)

One position layer per substitution type, one per deletions and one per insertion type

Ref. | A | A | C | T | G | G | A | T | T | C | G | | T | C

A | C | | | T | T | | | | | C | N | T | T

Read 1

| A | | 0 | 0 | | | | | | |
|---|---|---|---|---|---|---|---|---|---|
| C | | 2 | 0 | 6 | 0 | | | | |
| G | | 0 | 2 | 0 | | | | | |
| T | | 5 | 0 | 5 | 9 | 0 | | | |
| N | | 7 | 0 | | | | | | |
| del | | 3 | 0 | 3 | 0 | | | | |
| insA | | 0 | 7 | 0 | | | | | |
| insC | | 0 | 0 | | | | | | |
| insG | | 0 | 0 | | | | | | |
| insT | | 8 | 0 | | | | | | |

Read 2

| A-C mismatch | 2 |
|---|---|
| Deletion | 3 |
| G-T mismatch | 5 |
| G-N mismatch | 7 |
| Insert T | 8 |
| Now all read pointers are on a 0. They move to the next element and the smallest value is the first mismatch or indel | |
| C-G mismatch | 2 |
| Deletion | 3 |
| G-T mismatch | 5 |
| G-C mismatch | 6 |
| Insert A | 7 |
| C-T mismatch | 9 |

| A | G | | G | T | Read 3 | Read 4 | C | A | T | T

Ref. | A | A | C | T | G | G | A | T | T | C | G | | T | C

**Figure 18 - When no mismatches or indels of a given type are present for a read, a 0 is encoded in the corresponding layer. The 0 acts as reads separator and terminator in each layer.**

Reference sequence 1

| A | A | C | T | G | G | A | T | T | C | G | C | C | A |

| A | A | C | T | G | G |

| A | C | T | G | G | A |

| C | T | C | G | A | A |

Mismatching positions

| T | C | G | A | A | T |

} Class M reads with respect to ref. seq. 1

Reference sequence 2

| A | A | C | T | C | G | A | A | T | C | G | C | C | A |

↑

"Adapted" reference sequence

| C | T | C | G | A | A |

| T | C | G | A | A | T |

} Class P reads with respect to ref. seq. 2

**Figure 19 - A modification in the reference sequence can transform M reads in P reads.**

**Figure 20 - Genomic encoder.**

33

**Figure 21 – Genomic Decoder.**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150227686 A